# EUROPEAN PATENT SPECIFICATION

(11) **EP 1 083 874 B1**
(45) Date of publication and mention of the grant of the patent: **02.01.2004**
(21) Application number: 98925163.2
(22) Date of filing: 03.06.1998
(51) Int. Cl.: A61K 7/48, A61K 31/445

(54) **Use of a compound for the preparation of a medicament for treating alopecia or promoting hair growth in an animal**
Verwendung einer Verbindung zur Herstellung eines Medikaments zur Behandlung von Alopecia oder zur Förderung des Haarwuchses bei Tieren
Utilisation d'un composé pour la préparation d'un médicament pour le traitement de l' alopécie ou pour favoriser la croissance des poils chez les animaux

(43) Date of publication of application: 21.03.2001
(73) Proprietor: GPI NIL Holdings, Inc., Baltimore, MD 21224 (US)
(72) Inventor: HAMILTON, Gregory, S., Catonsville, MD 21228 (US); STEINER, Joseph, P., Mr. Airy, MD 21771 (US)
(74) Representative: HOFFMANN EITLE
(86) International application number: PCT/US1998/011264
(87) International publication number: WO 1999/062491

(56) References cited:
- EP-A- 0 423 714
- WO-A-98/13343

## Description

This application is a continuation-in-part of U.S. Patent Application No. 08/869,426, filed on June 4, 1997.

### BACKGROUND OF THE INVENTION

### 1. Field of Invention

This invention relates to pharmaceutical compositions and methods for treating alopecia and promoting hair growth using low molecular weight, small molecule pipecolic acid derivatives.

### 2. Description of Related Art

Hair loss occurs in a variety of situations. These situations include male pattern alopecia, alopecia senilis, alopecia areata, diseases accompanied by basic skin lesions or tumors, and systematic disorders such as nutritional disorders and internal secretion disorders. The mechanisms causing hair loss are very complicated, but in some instances can be attributed to aging, genetic disposition, the activation of male hormones, the loss of blood supply to hair follicles, and scalp abnormalities.

The immunosuppressant drugs FK506, rapamycin and cyclosporin are well known as potent T-cell specific immunosuppressants, and are effective against graft. rejection after organ transplantation. It has been reported that topical, but not oral, application of FK506 (Yamamoto et al., J. Invest. Dermatol., 1994, 102, 160-164; Jiang et al., J. Invest. Dermatol. 1995, 104, 523-525) and cyclosporin (Iwabuchi et al., J. Dermatol. Sci. 1995, 9, 64-69) stimulates hair growth in a dose-dependent manner. One form of hair loss, alopecia areata, is known to be associated with autoimmune activities; hence, topically administered immunomodulatory compounds are expected to demonstrate efficacy for treating that type of hair loss. The hair growth stimulating effects of FK506 have been the subject of an international patent filing covering FK506 and structures related thereto for hair growth stimulation (Honbo et al., EP 0 423 714 A2). Honbo et al. discloses the use of relatively large tricyclic compounds, known for their immunosuppressive effects, as hair revitalizing agents.

The hair growth and revitalization effects of FK506 and related agents are disclosed in many U.S. patents (Goulet et al., U.S. Patent No. 5,258,389; Luly et al., U.S. Patent No. 5,457,111; Goulet et al., U.S. Patent No. 5,532,248; Goulet et al., U.S. Patent No. 5,189,042; and Ok et al., U.S. Patent No. 5,208,241; Rupprecht et al., U.S. Patent No. 5,284,840; Organ et al., U.S. Patent No. 5,284,877). These patents claim FK506 related compounds. Although they do not claim methods of hair revitalization, they disclose the known use of FK506 for effecting hair growth. Similar to FK506 (and the claimed variations in the Honbo et al. patent), the compounds claimed in these patents are relatively large. Further, the cited patents relate to immunomodulatory compounds for use in autoimmune related diseases, for which FK506's efficacy is well known.

Other U.S. patents disclose the use of cyclosporin and related compounds for hair revitalization (Hauer et al., U.S. Patent No. 5,342,625; Eberle, U.S. Patent No. 5,284,826; Hewitt et al., U.S. Patent No. 4,996,193). These patents also relate to compounds useful for treating autoimmune diseases and cite the known use of cyclosporin and related immunosuppressive compounds for hair growth.

However, immunosuppressive compounds by definition suppress the immune system and also exhibit other toxic side effects. Accordingly, there is a need for non-immunosuppressant, small molecule compounds which are useful as hair revitalizing compounds.

Hamilton and Steiner disclose in U.S. Patent No. 5,614,547 novel pyrrolidine carboxylate compounds which bind to the immunophilin FKBP12 and stimulate nerve growth, but which lack immunosuppressive effects. Unexpectedly, it has been discovered that these non-immunosuppressant compounds promote hair growth with an efficacy similar to FK506. Yet their novel small molecule structure and non-immunosuppressive properties differentiate them from FK506 and related immunosuppressive compounds found in the prior art.

Document WO 98/13343 discloses neurotrophic low molecular weight small molecule heterocyclic thioesters and ketones having an affinity for FKBP-type immunophihins, and their use as inhibition of the enzyme activity associated with immunophilin proteins.

### SUMMARY OF THE INVENTION

The present invention relates to the use of a compound for the preparation of a medicament for treating alopecia or promoting hair growth in an animal, which comprises administering to said animal an effective amount of a low molecular weight, small molecule of a compound according to formula I 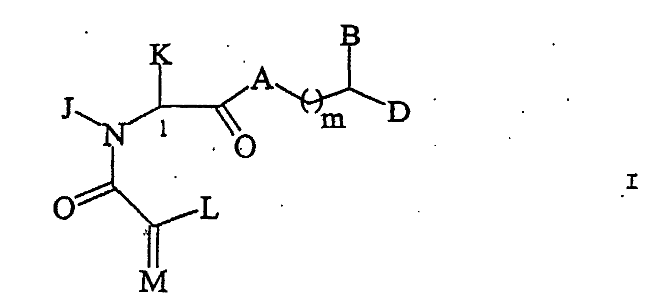 or a pharmaceutically acceptable salt, ester, or solvate thereof, wherein:
A is O, NH, or N-(C₁-C₄ alkyl);
B and D are independently Ar, C₅-C₇ cycloalkyl substituted C₁-C₆ straight or branched chain alkyl or C₂-C₆ straight or branched chain alkenyl, C₅-C₇ cycloalkenyl substituted C₁-C₆ straight or branched chain alkyl or C₂-C₆ straight or branched chain alkenyl, or Ar substituted C₁-C₆ straight or branched chain alkyl or C₂-C₆ straight or branched chain alkenyl,
   wherein in each case, one or two carbon atom(s) of said alkyl or alkenyl is/are optionally substituted with one or two heteroatom(s) independently selected from the group consisting of oxygen, sulfur, SO, and SO₂,
   or B and D are independently the fragment 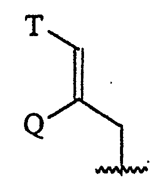 wherein Q is hydrogen, C₁-C₆ straight or branched chain alkyl or C₂-C₆ straight or branched chain alkenyl,
   and wherein T is Ar or C₅-C₇ cycloalkyl substituted at positions 3 and 4 with substituents independently selected from the group consisting of hydrogen, hydroxy, O-(C₁-C₄ alkyl) , O-(C₂-C₄ alkenyl), and carbonyl;
   Ar is selected from the group consisting of 1-napthyl, 2-napthyl, 2-furyl, 3-furyl, 2-thienyl, 3-thienyl, 2-pyridyl, 3-pyridyl, 4-pyridyl, phenyl, and monocyclic and bicyclic heterocyclic ring systems with individual ring sizes being 5 or 6 which have in either or both rings a total of 1-4 heteroatoms independently selected from oxygen, nitrogen and sulfur,
   wherein Ar has 1-3 substituent(s) independently selected from the group consisting of hydrogen, halo, hydroxy, hydroxymethyl, nitro, CF₃, trifluoromethoxy, C₁-C₆ straight or branched chain alkyl, C₂-C₆ C₁-C₆ straight or branched chain alkyl, C₂-C₆ straight or branched chain alkenyl, O-(C₁-C₄ straight or branched chain alkyl), O-(C₂-C₄ straight or branched chain alkenyl), O-benzyl, O-phenyl, amino, 1,2-methylenedioxy, carbonyl, and phenyl;
L is either hydrogen or U, and M is either oxygen or CH-U,
   provided that if L is hydrogen, then M is CH-U, or if M is oxygen then L is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇ cycloalkyl, C₅-C₇ cycloalkenyl substituted with C₁-C₄ straight or branched chain alkyl or C₂-C₄ straight or branched chain alkenyl, (C₁-C₄ alkyl or C₂-C₄ alkenyl)-Ar, or Ar;
J is hydrogen, C₁ or C₂ alkyl, or benzyl; K is C₁-C₄ straight or branched chain alkyl, benzyl or cyclohexylmethyl; or J and K are taken together to form a 7 membered heterocyclic ring which is substituted with oxygen, sulfur, SO, or SO₂; and
m is 0-3.

The present invention further relates to a pharmaceutical composition which comprises:
(i) an effective amount of a compound according to formula I; and
(ii) a pharmaceutically acceptable carrier.

The compounds used in the pharmaceutical compositions include immunosuppressive and non-immunosuppressive compounds having an affinity for FKBP-type immunophilins, particularly FKBP12. Non-immuno-suppressive compounds, as their name suggests, do not exert any significant immunosuppressive activity.

### BRIEF DESCRIPTION OF THE DRAWINGS

FIG. 1 is a photograph of mice treated with a vehicle after six weeks. FIG. 1 shows that less than 3% of the shaved area is covered with new hair growth when the vehicle (control) is administered.
FIG. 2 is a photograph of mice treated with 10 µM of GPI 1044 after six weeks. FIG. 2 shows that 90% of the shaved area is covered with new hair growth when GPI 1044 is administered.
FIG. 3 is a bar graph plotting the hair growth scores of unshaven animals and shaven animals treated with a vehicle, GPI 1044 (1 µM, 3 µM and 10 µM), and related pipecolic acid derivative neuroimmunophilin FKBP ligands GPI 1116 (1 µM and 10 µM) and GPI 1102 (1 µM and 3 µM).
FIG. 4 is a bar graph depicting the relative hair growth indices for C57 Black 6 mice treated with a vehicle, FK506, and related neuroimmunophilin FKBP ligands 14 days after treatment with each identified compound. Figure 4 demonstrates the remarkable early hair growth promoted by neuroimmunophilin FKBP ligands.

### DETAILED DESCRIPTION OF THE INVENTION

### Definitions

"Alopecia" refers to deficient hair growth and partial or complete loss of hair, including without limitation androgenic alopecia (male pattern baldness), toxic alopecia, alopecia senilis, alopecia areata, alopecia pelada and trichotillomania. Alopecia results when the pilar cycle is disturbed. The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae, and the hairs fall out. Alopecia has a number of etiologies, including genetic factors, aging, local and systemic diseases, febrile conditions, mental stresses, hormonal problems, and secondary effects of drugs.

"GPI 1044" refers to Compound 4.

"GPI 1102" refers to 4-phenyl-1-(3-phenylpropyl) butyl 1-(3,3-dimethyl-2-oxopentanoyl)-2-piperidinecarboxylate.

"GPI 1116" refers to 1-phenethyl-3-phenylpropyl 1-(3,3-dimethyl-2-oxopentanoyl)-2-piperidinecarboxylate.

"GPI 1206" refers to a compound of formula

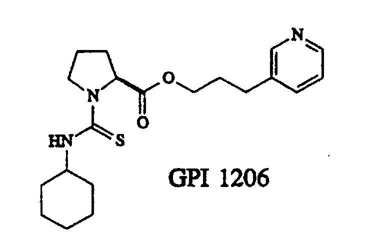

"Isomers" refer to different compounds that have the same molecular formula. "Stereoisomers" are isomers that differ only in the way the atoms are arranged in space. "Enantiomers" are a pair of stereoisomers that are non-superimposable mirror images of each other. "Diastereoisomers" are stereoisomers which are not mirror images of each other. "Racemic mixture" means a mixture containing equal parts of individual enantiomers. "Non-racemic mixture" is a mixture containing unequal parts of individual enantiomers or stereoisomers.

"Pharmaceutically acceptable salt, ester, or solvate" refers to a salt, ester, or solvate of a subject compound which possesses the desired pharmacological activity and which is neither biologically nor otherwise undesirable. A salt, ester, or solvate can be formed with inorganic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride, hydrobromide, hydroiodide, 2-hydroxyethanesulfonate, lactate, maleate, methanesulfonate, naphthylate, 2-naphthalenesulfonate, nicotinate, oxalate, sulfate, thiocyanate, tosylate and undecanoate. Examples of base salts, esters, or solvates include ammonium salts; alkali metal salts, such as sodium and potassium salts; alkaline earth metal salts, such as calcium and magnesium salts; salts with organic bases, such as dicyclohexylamine salts; N-methyl-D-glucamine; and salts with amino acids, such as arginine, lysine, and so forth. Also, the basic nitrogen-containing groups can be quarternized with such agents as lower alkyl halides, such as methyl, ethyl, propyl, and butyl chlorides, bromides, and iodides; dialkyl sulfates, such as dimethyl, diethyl, dibutyl, and diamyl sulfates; long chain halides, such as decyl, lauryl, myristyl, and stearyl chlorides, bromides, and iodides; aralkyl halides, such as benzyl and phenethyl bromides; and others. Water or oil-soluble or dispersible products are thereby obtained.

"Pilar cycle" refers to the life cycle of hair follicles, and includes three phases:
(1) the anagen phase, the period of active hair growth which, insofar as scalp hair is concerned lasts about three to five years;
(2) the catagen phase, the period when growth stops and the follicle atrophies which, insofar as scalp hair is concerned, lasts about one to two weeks; and
(3) the telogen phase, the rest period when hair progressively separates and finally falls out which, insofar as scalp hair is concerned, lasts about three to four months .
Normally 80 to 90 percent of the follicles are in the anagen phase, less than 1 percent being in the catagen phase, and the rest being in the telogen phase. In the telogen phase, hair is uniform in diameter with a slightly bulbous, non-pigmented root. By contrast, in the anagen phase, hair has a large colored bulb at its root.

"Promoting hair growth" refers to maintaining, inducing, stimulating, accelerating, or revitalizing the germination of hair.

"Treating alopecia" refers to:
(i) preventing alopecia in an animal which may be predisposed to alopecia; and/or
(ii) inhibiting, retarding or reducing alopecia; and/or
(iii) promoting hair growth; and/or
(iv) prolonging the anagen phase of the hair cycle; and/or
(v) converting vellus hair tc growth as terminal hair. Terminal hair is coarse, pigmented, long hair in which the bulb of the hair follicle is seated deep in the dermis. Vellus hair, on the other hand, is fine, thin, non-pigmented short hair in which the hair bulb is located superficially in the dermis. As alopecia progresses, the hairs change from the terminal to the vellus type.

### The use of the Present Invention

The present invention relates to the use of a compound for the preparation of a medicament for treating alopecia or promoting hair growth in an animal, which comprises administering to said animal an effective amount of said compound.

The compound is particularly useful for treating male pattern alopecia, alopecia senilis, alopecia areata, alopecia resulting from skin lesions or tumors, alopecia resulting from cancer therapy such as chemotherapy and radiation, and alopecia resulting from systematic disorders such as nutritional disorders and internal secretion disorders.

### Pharmaceutical Compositions of the Present Invention

The present invention also relates to a pharmaceutical composition comprising:
(i) an effective amount of a compound for treating alopecia or promoting hair growth in an animal; and
(ii) a pharmaceutically acceptable carrier.

The compounds used in the pharmaceutical compositions of the present invention are low molecular weight, small molecule compounds having an affinity for FKBP-type immunophilins, such as FKBP12. When the compound binds to an FKBP-type immunophilin, it has been found to inhibit the prolyl-peptidyl cis-transisomerase, or rotamase, activity of the binding protein. Unexpectedly, the compounds have also been found to stimulate hair growth. These rotamase inhibiting compounds may be immunosuppressive or non-immunosuppressive. Examples of useful compounds are set forth below.

Representative species of Formula I are presented in Table I.

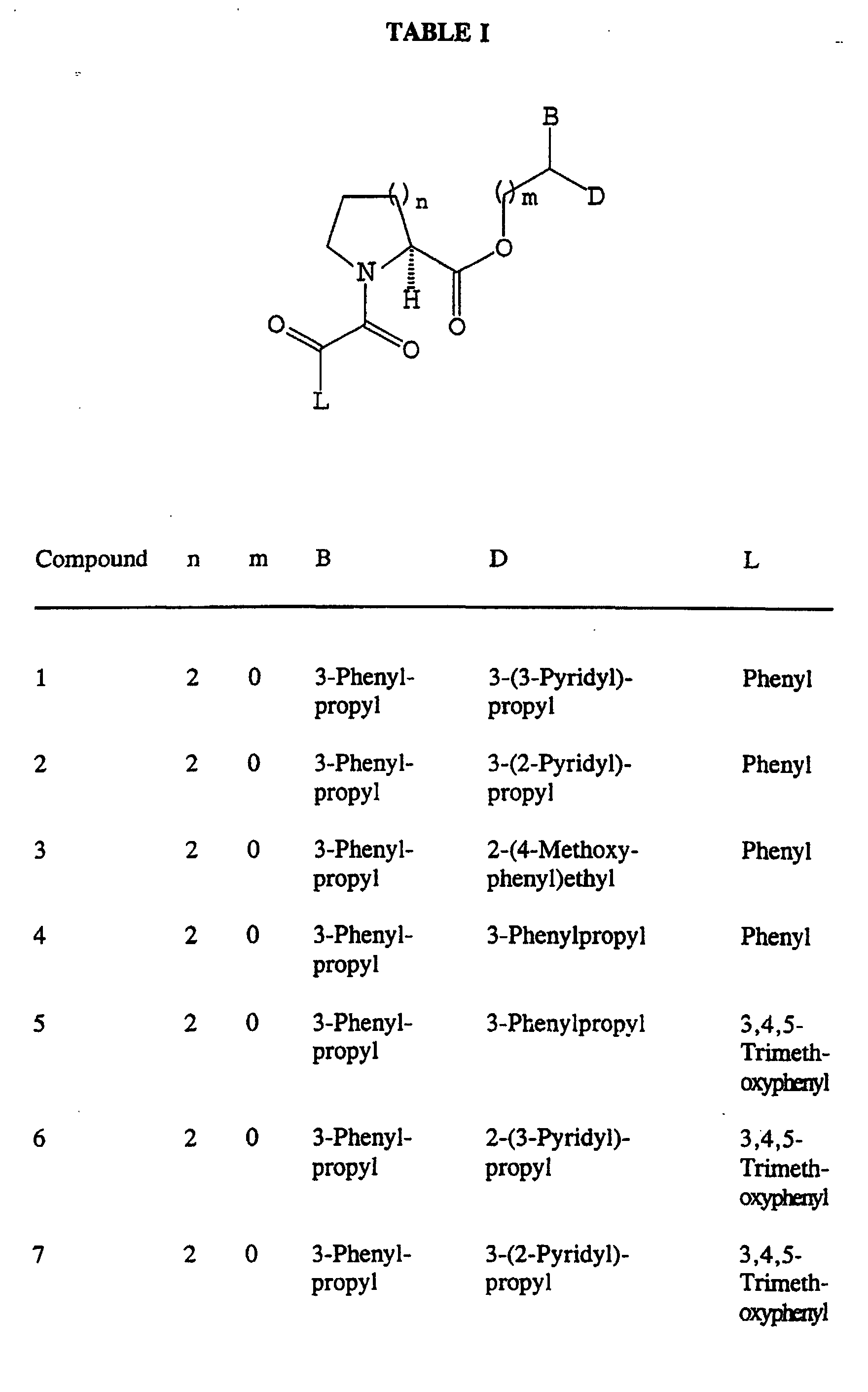

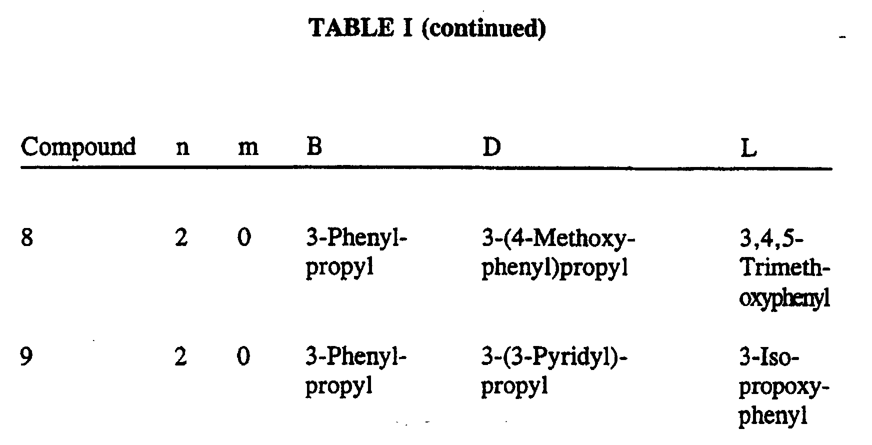

A specific compound of formula I is represented by formula II. 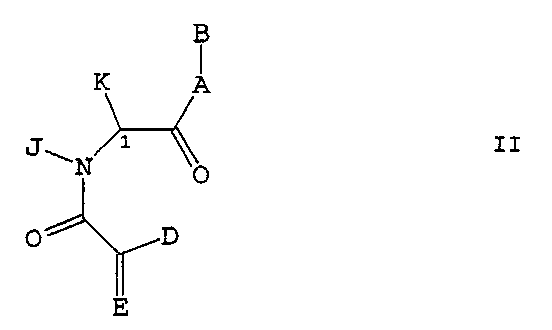 or a pharmaceutically acceptable salt, ester, or solvate thereof, wherein:
A is O, NH, or N-(C₁-C₄ alkyl) ;
B is hydrogen, CHL-Ar, C₁-C₆ straight or branched chain alkyl, C₂-C₆ straight or branched chain alkenyl, C₅-C₇ cycloalkyl, C₅-C₇ cycloalkenyl, Ar substituted C₁-C₆ alkyl or C₂-C₆ alkenyl, or 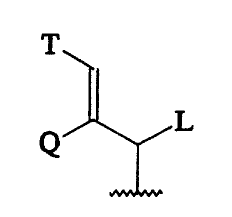 wherein L and Q are independently hydrogen, C₁-C₆ straight or branched chain alkyl, or C₂-C₆ straight or branched chain alkenyl; and
   T is Ar or C₅-C₇ cyclohexyl substituted at positions 3 and 4 with substituents independently selected from the group consisting of hydrogen, hydroxy, O-(C₁-C₄ alkyl), O-(C₂-C₄ alkenyl), and carbonyl;
Ar is selected from the group consisting of 1-napthyl, 2-napthyl, 2-furyl, 3-furyl, 2-thienyl, 2-pyridyl, 3-pyridyl, 4-pyridyl and phenyl having 1-3 substituent(s) independently selected from the group consisting of hydrogen, halo, hydroxy, nitro, CF₃, C₁-C₆ straight or branched chain alkyl, C₂-C₆ straight or branched chain alkenyl, O-(C₁-C₄ straight or branched chain alkyl), O-(C₂-C₄ straight or branched chain alkenyl), O-benzyl, O-phenyl, amino, and phenyl.
D is hydrogen or U; E is oxygen or CH-U, provided that if D is hydrogen, then E is CH-U, or if E is oxygen, then D is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇-cycloalkyl, C₅-C₇ cycloalkenyl substituted with C₁-C₄ straight or branched chain alkyl or C₂-C₄ straight or branched chain alkenyl, 2-indolyl, 3-indolyl, (C₁-C₄ alkyl or C₂-C₄ alkenyl)-Ar, or Ar;
J is hydrogen, C₁ or C₂ alkyl, or benzyl; K is C₁-C₄ straight or branched chain alkyl, benzyl or cyclohexylethyl; or J and K are taken together to form a 5-7 membered heterocyclic ring which is substituted with oxygen, sulfur, SO, or SO₂.

A preferred compound of formula I is a compound of Formula III 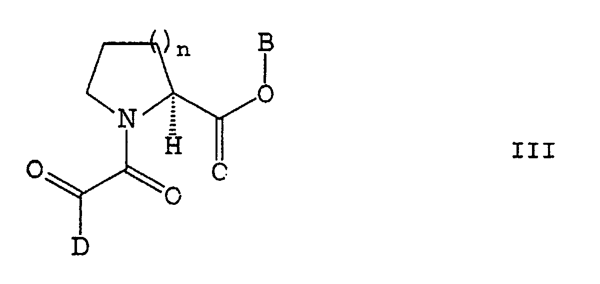 or a pharmaceutically acceptable salt, ester, or solvate thereof, wherein:
n is 2;
D is phenyl, methoxy, 2-furyl, or 3,4,5-trimethoxyphenyl; and
B is benzyl, 3-phenylpropyl, 4-(4-methoxyphenyl)butyl, 4-phenylbutyl, phenethyl, 3-cyclohexylpropyl, 4-cyclohexylbutyl, 3-cyclopentylpropyl, 4-cyclohexylbutyl, 3-phenoxybenzyl, 3-(3-indolyl)propyl, or 4-(4-methoxyphenyl)butyl;
   provided that:
   when D is phenyl, then B is benzyl, 3-phenylpropyl, 4-(4-methoxyphenyl)butyl, 4-phenylbutyl, phenethyl, or 4-cyclohexylbutyl;
   when D is methoxy, B is benzyl, 4-cyclohexylbutyl, 3-cyclohexylpropyl, or 3-cyclopentylpropyl;
   when D is 2-furyl, then B is benzyl; and
   when D is 3,4,5-trimethoxyphenyl, then B is 4-cyclohexylbutyl, 3-phenoxybenzyl, 4-phenylbutyl, 3-(3-indolyl)propyl, or 4-(4-methoxyphenyl)butyl.

Representative species of Formula III are presented in Table II.

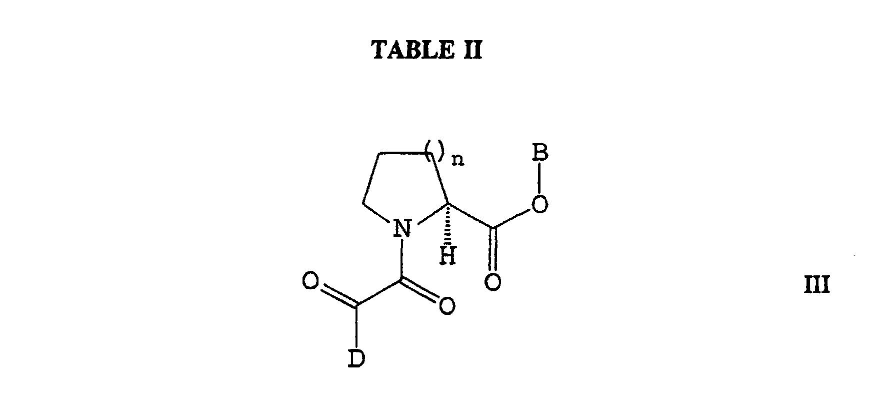

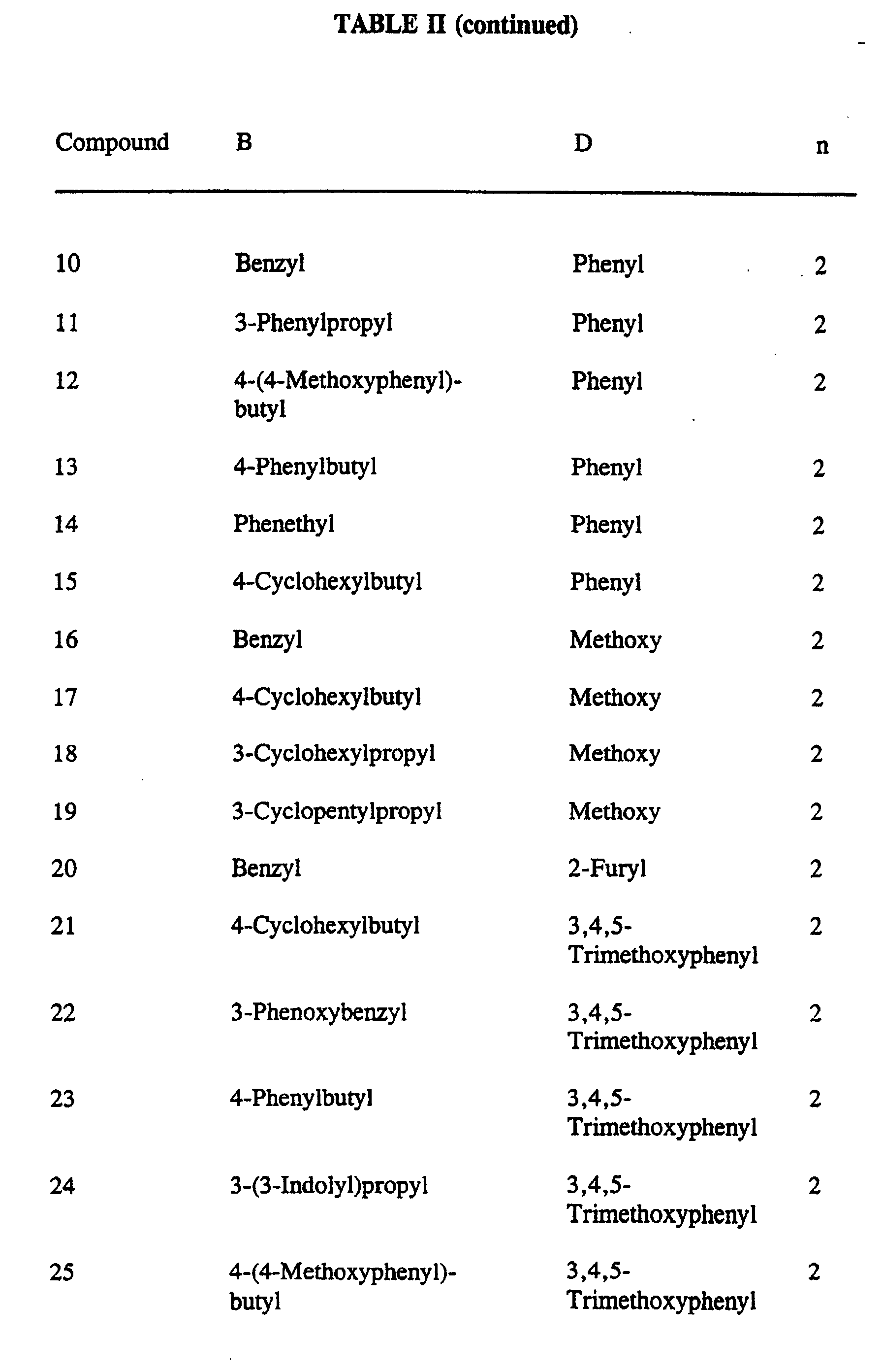

A preferred compound of formula I is a compound of formula IV 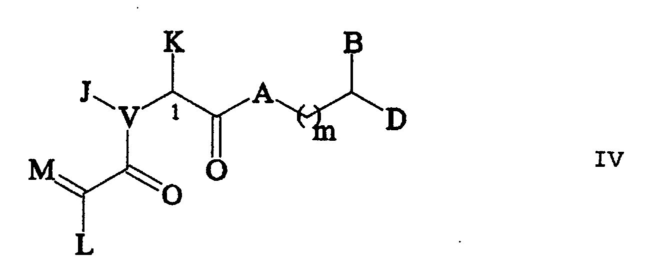 or a pharmaceutically acceptable salt, ester, or solvate thereof, wherein:
V is C, N, or S;
J and K, taken together with V and the carbon atom to which they are respectively attached, form a 5-7 membered saturated or unsaturated heterocyclic ring containing, in addition to V, one or more heteroatom(s) selected from the group consisting of O, S, SO, SO₂, N, NH, and NR;
R is either C₁-C₉ straight or branched chain alkyl, C₂-C₉ straight or branched chain alkenyl, C₃-C₉ cycloakyl, C₅-C₇ cycloalkenyl, or Ar₁, wherein R is either unsubstituted of substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₂;
Ar₁ and Ar₂ are independently an alicyclic or aromatic, mono-, bi- or tricyclic, carbo- or heterocyclic ring; wherein the individual ring size is 5-8 members; wherein said heterocyclic ring contains 1-6 heteroatom(s) independently selected from the group consisting of 0, N, and S;
A, B, D, L, M, and m are as defined in Formula I above; and
said pipecolic acid derivative has an affinity for FKBP-type immunophilins.

All the compounds of Formulas I-IV possess asymmetric centers and thus can be produced as mixtures of stereoisomers or as individual R- and S-stereoisomers. The individual stereoisomers may be obtained by using an optically active starting material, by resolving a racemic or non-racemic mixture of an intermediate at some appropriate stage of the synthesis, or by resolving the compounds of Formulas I-IV. It is understood that the compound cf Formulas I-IV encompass individual stereoisomers as well as mixtures (racemic and non-racemic) of stereoisomers. Preferably, S-stereoisomers are used in the pharmeceutical compositions of the present invention.

### Affinity for FKBP12

The compounds used in the pharmaceutical compositions have an affinity for the FK506 binding protein, particularly FKBP12. The inhibition of the prolyl peptidyl *cis-trans* isomerase activity of FKBP may be measured as an indicator of this affinity.

### Kᵢ Test Procedure

Inhibition of the peptidyl-prolyl isomerase (rotamase) activity of the compounds used in the pharmaceutical compositions can be evaluated by known methods described in the literature (Harding et al., *Nature*, 1989, 341:758-760; Holt et al. *J. Am. Chem. Soc*., 115:9923-9938). These values are obtained as apparent Kᵢ's and are presented for representative compounds in TABLE III.

The cis-trans isomerization of an alanine-proline bond in a model substrate, N-succinyl-Ala-Ala-Pro-Phe-*p*-nitroanilide, is monitored spectrophotometrically in a chymotrypsin-coupled assay, which releases *para*-nitroanilide from the trans form of the substrate. The inhibition of this reaction caused by the addition of different concentrations of inhibitor is determined, and the data is analyzed as a change in first-order rate constant as a function of inhibitor concentration to yield the apparent Kᵢ values.

In a plastic cuvette are added 950 mL of ice cold assay buffer (25 mM HEPES, pH 7.8, 100 mM NaCl), 10 mL of FKBP (2.5 mM in 10 mM Tris-Cl pH 7.5, 100 mM NaCl, 1 mM dithiothreitol), 25 mL of chymotrypsin (50 mg/ml in 1 mM HCl) and 10 mL of test compound at various concentrations in dimethyl sulfoxide. The reaction is initiated by the addition of 5 mL of substrate (succinyl-Ala-Phe-Pro-Phe-*para*-nitroanilide, 5 mg/mL in 2.35 mM LiCl in trifluoroethanol).

The absorbance at 390 nm versus time is monitored for 90 seconds using a spectrophotometer and the rate constants are determined from the absorbance versus time data files.

**TABLE III**

| ***In Vitro* Test Results - Formulas I-III** | |
|---|---|
| **Compound** | **K**_{**i**} **(µM)** |
| 10 | 1.5 |
| 13 | 0.35 |
| 14 | 1.1 |
| 15 | 0.4 |
| 16 | 80 |
| 17 | 6 |
| 18 | 20 |
| 19 | 35 |
| 20 | 3 |
| 21 | 0.04 |
| 22 | 0.018 |
| 23 | 0.019 |
| 24 | 0.017 |
| 25 | 0.013 |

### Route of Administration

To effectively treat alopecia or promote hair growth, the compounds used in the pharmaceutical compositions must readily affect the targeted areas. For these purposes, the compounds are preferably administered topically to the skin.

For topical application to the skin, the compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water. Alternatively, the compounds can be formulated into suitable lotions or creams containing the active compound suspended or dissolved in, for example, a mixture of one or more of the following: mineral oil, sorbitan monostearate, polysorbate 60, cetyl ester wax, cetearyl alcohol, 2-octyldodecanol, benzyl alcohol and water.

Other routes of administration known in the pharmaceutical art are also contemplated by this invention.

### Dosage

Dosage levels on the order of about 0.1 mg to about 10,000 mg of the active ingredient compound are useful in the treatment of the above conditions, with preferred levels of about 0.1 mg to about 1,000 mg. The specific dose level for any particular patient will vary depending upon a variety of factors, including the activity of the specific compound employed; the age, body weight, general health, sex and diet of the patient; the time of administration; the rate of excretion; drug combination; the severity of the particular disease being treated; and the form of administration. Typically, *in vitro* dosage-effect results provide useful guidance on the proper doses for patient administration. Studies in animal models are also helpful. The considerations for determining the proper dose levels are well known in the art.

The compounds can be administered with other hair revitalizing agents. Specific dose levels for the other hair revitalizing agents will depend upon the factors previously stated and the effectiveness of the drug combination.

### EXAMPLES

The following examples are illustrative of the present invention and are not intended to be limitations thereon. Unless otherwise indicated, all percentages are based upon 100% by weight of the final composition.

### Example 1

### In Vivo Hair Generation Tests With C57 Black 6 Mice

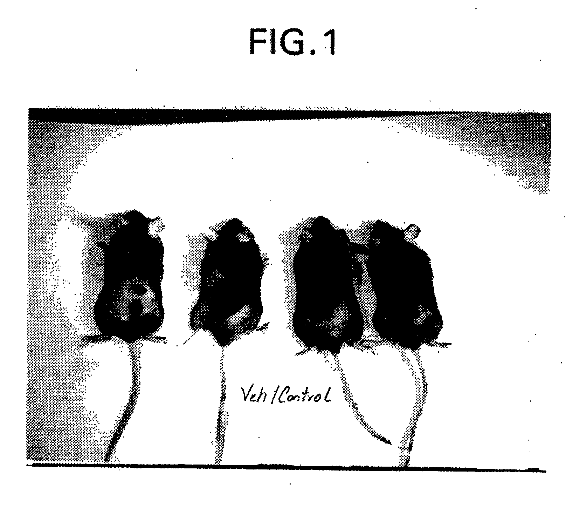

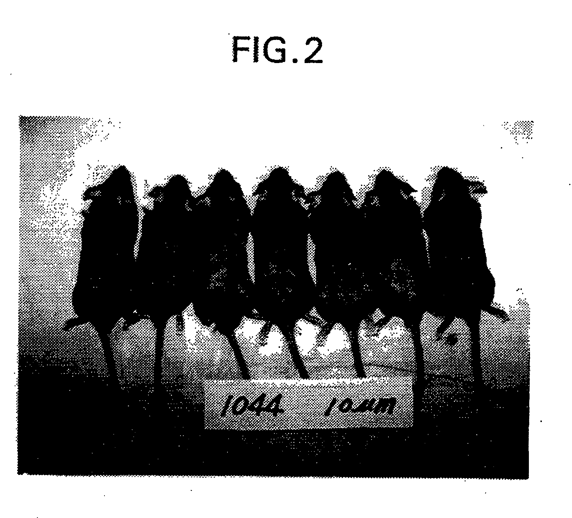

Experiment A: C57 black 6 mice were used to demonstrate the hair revitalizing properties of GPI 1044 (compound 4), as well as neuroimmunophilin FKBP ligands GPI 1102 and GPI 1116. C57 black 6 mice, approximately 7 weeks old, had an area of 5,08 cm (2 inches) by 5,08 cm (2 inches) on their hindquarters shaved to remove all existing hair. Care was taken not to nick or cause abrasion to the underlaying dermal layers. The animals were in anagen growth phase, as indicated by the pinkish color of the skin. Referring now to FIGS. 1 and 2, four animals were treated by topical administration with 20% propylene glycol vehicle (FIG. 1), and seven animals were treated by topical administration with 10 µM GPI 1044 (FIG. 2). The animals were treated with vehicle or GPI 1044 every 48 hours (3 applications total over the course of 5 days) and the hair growth was allowed to proceed for 6 weeks. Hair growth was quantitated by the percent of shaved area covered by new hair growth during this time period.

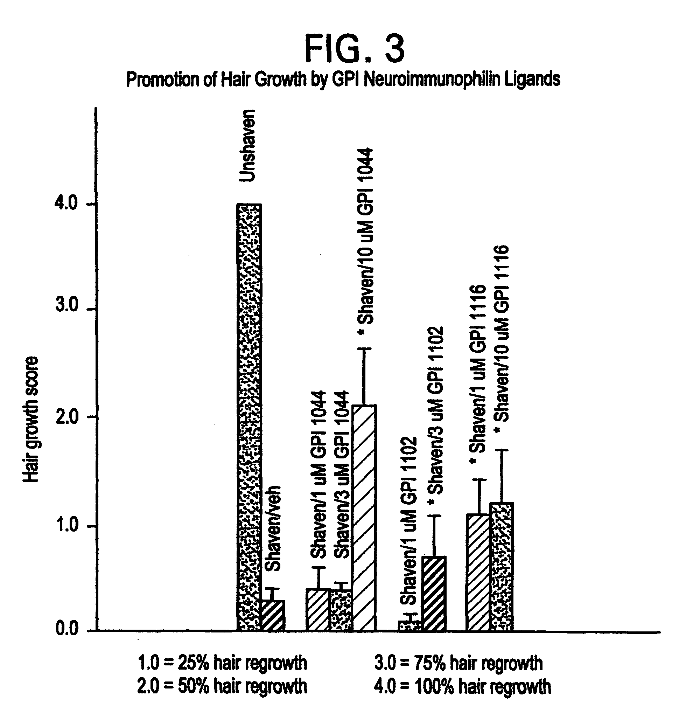

FIG. 1 shows that animals treated with vehicle exhibited only a small amount of hair growth in patches or tufts, with less than 3% of the shaved area covered with new growth. In contrast, FIGS. 2 shows that animals treated with 10 µM GPI 1044 exhibited dramatic hair growth, covering as much as 50% of the shaved area in some animals. FIG. 3 compares the hair growth score of unshaven animals with the hair growth scores of shaven animals treated with a vehicle and GPI 1044 (1 µM, 3 µM and 10 µM), as well as related neuroimmunophilin FKBP ligands GPI 1116 (1 µM and 10 µM) and GPI 1102 (1 µM and 3 µM).

Experiment B: C57 Black 6 mice were used to demonstrate the hair revitalizing properties of neuroimmunophilin FKBP ligands. C57 Black 6 mice, 55 to 75 days old, had an area of 5,08 an (2 inches) by (2) 5,08 cm (inches) on their hindquarters shaved to remove all existing hair. Care was taken not to nick or cause abrasion to the underlying dermal layers. The animals were in a anagen growth phase when shaved. Five animals per group were treated by topical administration with a vehicle, FK506, or a neuroimmunophilin FKBP ligand (GPI 1116 or 1206) at a concentration of one micromole per milliliter to the shaved area. The animals were treated three times per week, and hair growth was evaluated 14 days after initiation of treatment. Hair growth was quantitated by the percent of shaved area covered by new hair growth, as scored by a blinded observer, on a scale of 0 (no growth) to five (complete hair regrowth in shaved area).

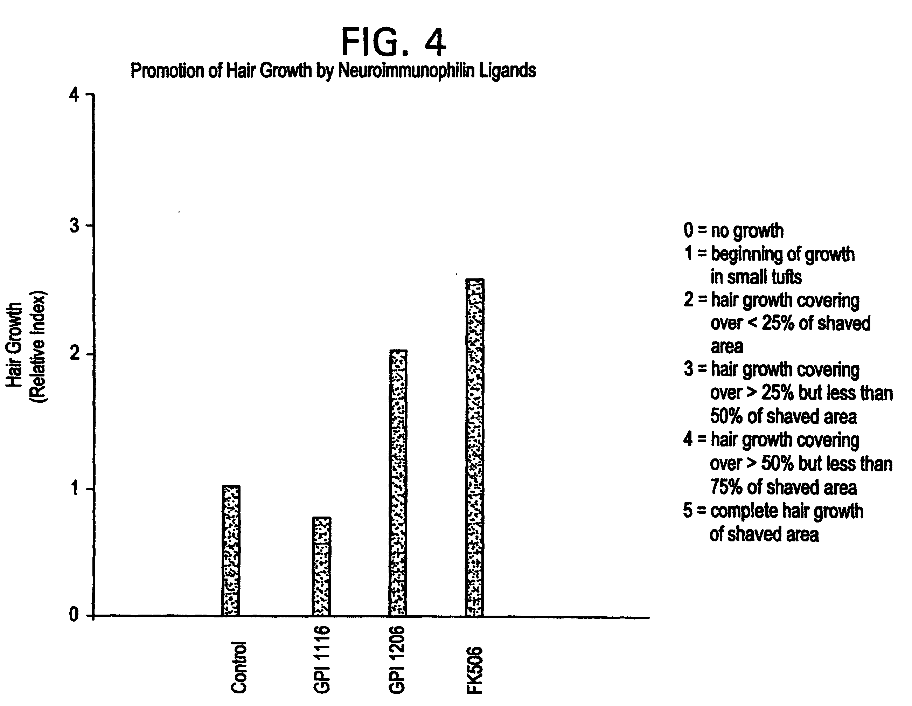

Figure 4 shows that after 14 days, the animals treated with vehicle exhibited the beginning of growth in small tufts. In contrast, animals treated with one of the low molecular weight, small molecule, neuroimmunophilin FKBP ligands exhibited dramatic hair growth.

### Example 2

A lotion comprising the following composition may be prepared.

| | (%) |
|---|---|
| 95% Ethanol | 80.0 |
| compound 4 | 10.0 |
| α-Tocopherol acetate | 0.01 |
| Ethylene oxide (40 mole) adducts of hardened castor oil | 0.5 |
| purified water | 9.0 |
| perfume and dye | q.s. |

Into 95% ethanol are added compound 4, α-tocopherol acetate, ethylene oxide (40 mole) adducts of hardened castor oil, perfume and a dye. The resulting mixture is stirred and dissolved, and purified water is added to the mixture to obtain a transparent liquid lotion.

5 ml of the lotion may be applied once or twice per day to a site having marked baldness or alopecia.

### Example 3

A lotion comprising the following composition shown may be prepared.

| | (%) |
|---|---|
| 95% Ethanol | 80.0 |
| compound 4 | 0.005 |
| Hinokitol | 0.01 |
| Ethylene oxide (40 mole) adducts of hardened castor oil | 0.5 |
| Purified water | 19.0 |
| Perfume and dye | q.s. |

Into 95% ethanol are added compound 4, hinokitol, ethylene oxide (40 mole) adducts of hardened castor oil, perfume, and a dye. The resulting mixture is stirred, and purified water is added to the mixture to obtain a transparent liquid lotion.

The lotion may be applied by spraying once to 4 times per day to a site having marked baldness or alopecia.

### Example 4

An emulsion may be prepared from A phase and B phase having the following compositions.

| **(A phase)** | **(%)** |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitan monooleate | 1.0 |
| compound 4 | 0.01 |

| **(B phase)** | **(%)** |
|---|---|
| Glycerine | 10.0 |
| Purified water | 69.0 |
| Perfume, dye, and preservative | q.s. |

The A phase and the B phase are respectively heated and melted and maintained at 80°C. Both phases are then mixed and cooled under stirring to normal temperature to obtain an emulsion.

The emulsion may be applied by spraying once to four times per day to a site having marked baldness or alopecia.

### Example 5

A cream may be prepared from A phase and B phase having the following compositions.

| **(A Phase)** | **(%)** |
|---|---|
| Fluid paraffin | 5.0 |
| Cetostearyl alcohol | 5.5 |
| Petrolatum | 5.5 |
| Glycerine monostearate | 33.0 |
| Polyoxyethylene (20 mole) 2-octyldodecyl ether | 3.0 |
| Propylparaben | 0.3 |

| **(B Phase)** | **(%)** |
|---|---|
| compound 4 | 0.8 |
| Glycerine | 7.0 |
| Dipropylene glycol | 20.0 |
| Polyethylene glycol 4000 | 5.0 |
| Sodium Hexametaphosphate | 0.005 |
| Purified water | 44.895 |

The A phase is heated and melted, and maintained at 70°C. The B phase is added into the A phase and the mixture is stirred to obtain an emulsion. The emulsion is then cooled to obtain a cream.

The cream may be applied once to 4 times per day to a site having marked baldness or alopecia.

### Example 6

A liquid comprising the following composition may be prepared.

| | (%) |
|---|---|
| Polyoxyethylene butyl ether | 20.0 |
| Ethanol | 50.0 |
| compound 4 | 0.001 |
| Propylene glycol | 5.0 |
| Polyoxyethylene hardened castor oil derivative (ethylene oxide 80 mole adducts) | 0.4 |
| Perfume | q.s. |
| Purified water | q.s. |

Into ethanol are added polyoxypropylene butyl ether, propylene glycol, polyoxyethylene hardened castor oil, compound 4, and perfume. The resulting mixture is stirred, and purified water is added to the mixture to obtain a liquid.

The liquid may be applied once to 4 times per day to a site having marked baldness or alopecia.

### Example 7

A shampoo comprising the following composition may be prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| compound 4 | 5.0 |
| Ethanol | 2.0 |
| Perfume | 0.3 |
| Purified water | 69.7 |

Into 69.7 of purified water are added 5.0 g of sodium laurylsulfate, 5.0 g of triethanolamine laurylsulfate, 6.0 g of betaine lauryldimethylaminoacetate. Then a mixture obtained by adding 5.0 g of compound 4, 5.0 g of polyethylene glycol, and 2.0 g of ethylene glycol distearate to 2.0 g of ethanol, followed by stirring, and 0.3 g of perfume are successively added. The resulting mixture is heated and subsequently cooled to obtain a shampoo.

The shampoo may be used on the scalp once or twice per day.

### Example 8

A patient is suffering from alopecia senilis. A pharmaceutical composition comprising a compound of formula I, may be administered to the patient. Increased hair growth is expected to occur following treatment.

### Example 9

A patient is suffering from male pattern alopecia. A pharmaceutical composition comprising a compound of formula I, may be administered to the patient. Increased hair growth is expected to occur following treatment.

### Example 10

A patient is suffering from alopecia areata. A pharmaceutical composition comprising a compound of formula I, may be administered to the patient. Increased hair growth is expected to occur following treatment.

### Example 11

A patient is suffering from hair loss caused by skin lesions. A pharmaceutical composition comprising a compound of formula I, may be administered to the patient. Increased hair growth is expected to occur following treatment.

### Example 12

A patient is suffering from hair loss caused by tumors. A pharmaceutical composition comprising a compound of formula I, may be administered to the patient. Increased hair growth is expected to occur following treatment.

### Example 13

A patient is suffering from hair loss caused by a systematic disorder, such as a nutritional disorder or an internal secretion disorder. A pharmaceutical composition comprising a compound of formula I, may be administered to the patient. Increased hair growth is expected to occur following treatment.

### Example 14

A patient is suffering from hair loss caused by chemotherapy. A pharmaceutical composition comprising a compound of formula I, may be administered to the patient . Increased hair growth is expected to occur following treatment.

### Example 15

A patient is suffering from hair loss caused by radiation. A pharmaceutical composition comprising a compound of formula I, may be administered to the patient . Increased hair growth is _ expected to occur following treatment.

The invention being thus described, it will be obvious that the same may be varied in many ways. Such variations are not to be regarded as a departure from the scope of the invention and all such modifications are intended to be included within the scope of the following claims.

## Claims

1. Use of a compound for the preparation of a medicament for treating alopecia or promoting hair growth in an animal in need thereof, wherein said compound is of formula I 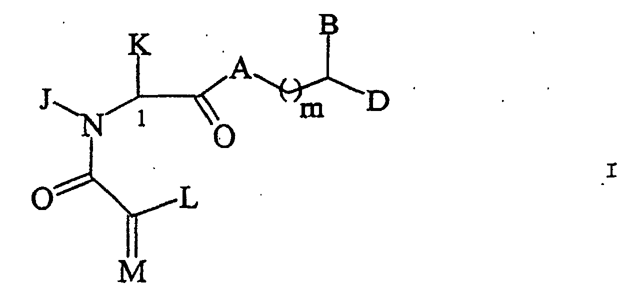 or a pharmaceutically acceptable salt, ester, or solvate thereof, wherein:
A is O, NH, or N-(C₁-C₄ alkyl);
B and D are independently Ar, C₅-C₇ cycloalkyl substituted C₁-C₆ straight or branched chain alkyl or C₂-C₆ straight or branched chain alkenyl, C₅-C₇ cycloalkenyl substituted C₁-C₆ straight or branched chain alkyl or C₂-C₆ straight or branched chain alkenyl, or Ar substituted C₁-C₆ straight or branched chain alkyl or C₂-C₆ straight or branched chain alkenyl,
wherein in each case, one or two carbon atom(s) of said alkyl or alkenyl is/are optionally substituted with one or two heteroatom(s) independently selected from the group consisting of oxygen, sulfur, SO, and SO₂,
or B and D are independently the fragment 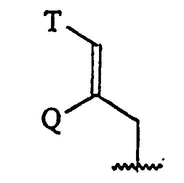 wherein Q is hydrogen, C₁-C₆ straight or branched chain alkyl or C₂-C₆ straight or branched chain alkenyl,
and wherein T is Ar or C₅-C₇ cycloalkyl substituted at positions 3 and 4 with substituents independently selected from the group consisting of hydrogen, hydroxy, O-(C₁-C₄ alkyl), O-(C₂-C₄ alkenyl), and carbonyl;
Ar is selected from the group consisting of 1-napthyl, 2-napthyl, 2-furyl, 3-furyl, 2-thienyl, 3-thienyl, 2-pyridyl, 3-pyridyl, 4-pyridyl, phenyl, and monocyclic and bicyclic heterocyclic ring systems with individual ring sizes being 5 or 6 which have in either or both rings a total of 1-4 heteroatoms independently selected from oxygen, nitrogen and sulfur,
wherein Ar has 1-3 substituent(s) independently selected from the group consisting of hydrogen, halo, hydroxy, hydroxymethyl, nitro, CF₃, trifluoromethoxy, C₁-C₆ straight or branched chain alkyl, C₂-C₆ straight or branched chain alkenyl, O-(C₁-C₄ straight or branched chain alkyl) , O-(C₂-C₄ straight or branched chain alkenyl), O-benzyl, O-phenyl, amino, 1,2-methylenedioxy, carbonyl, and phenyl;
L is either hydrogen or U, and M is either oxygen or CH-U,
provided that if L is hydrogen, then M is CH-U, or if M is oxygen then L is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇ cycloalkyl, C₅-C₇ cycloalkenyl substituted with C₁-C₄ straight or branched chain alkyl or C₂-C₄ straight or branched chain alkenyl, (C₁-C₄ alkyl or C₂-C₄ alkenyl)-Ar, or Ar;
J is hydrogen, C₁ or C₂ alkyl, or benzyl; K is C₁-C₄ straight or branched chain alkyl, benzyl or cyclohexylmethyl; or J and K are taken together to form a 7 membered heterocyclic ring which is substituted with oxygen, sulfur, SO, or SO₂; and
m is 0-3.

2. The use of claim 1, wherein said compound is of formula II 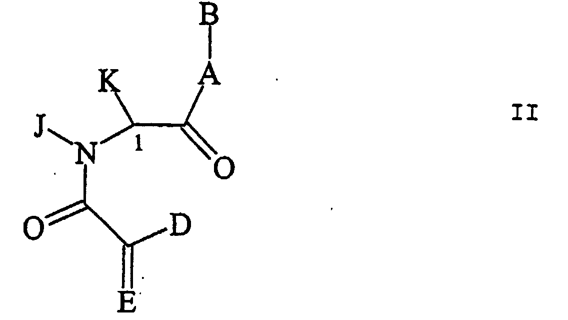 or a pharmaceutically acceptable salt, ester, or solvate thereof, wherein:
A is O, NH, or N-(C₁-C₄ alkyl);
B is hydrogen, CHL-Ar, C₁-C₆ straight or branched chain alkyl, C₂-C₆ straight or branched chain alkenyl, C₅-C₇ cycloalkyl, C₅-C₇ cycloalkenyl, Ar substituted C₁-C₆ alkyl or C₂-C₆ alkenyl, or 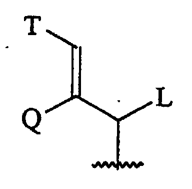 wherein L and Q are independently hydrogen, C₁-C₆ straight or branched chain alkyl, or C₂-C₆ straight or branched chain alkenyl,
and wherein T is Ar or C₅-C₇ cyclohexyl substituted at positions 3 and 4 with substituents independently selected from the group consisting of hydrogen, hydroxy, O-(C₁-C₄ alkyl), O-(C₂-C₄ alkenyl) , and carbonyl;
Ar is selected from the group consisting of 1-napthyl, 2-napthyl, 2-furyl, 3-furyl, 2-thienyl, 2-pyridyl, 3-pyridyl, 4-pyridyl, and phenyl,
wherein said Ar has 1-3 substituent(s) independently selected from the group consisting of hydrogen, halo, hydroxy, nitro, CF₃, C₁-C₆ straight or branched chain alkyl, C₂-C₆ straight or branched chain alkenyl, O-(C₁-C₄ straight or branched chain alkyl), O-(C₂-C₄ straight or branched chain alkenyl), O-benzyl, O-phenyl, amino, and phenyl;
D is hydrogen or U, and E is oxygen or CH-U, provided that if D is hydrogen, then E is CH-U, or if E is oxygen, then D is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇-cycloalkyl, C₅-C₇ cycloalkenyl substituted with C₁-C₄ straight or branched chain alkyl or C₂-C₄ straight or branched chain alkenyl, 2-indolyl, 3-indolyl, (C₁-C₄ alkyl or C₂-C₄ alkenyl)-Ar, or Ar; and
J is hydrogen, C₁ or C₂ alkyl, or benzyl; K is C₁-C₄ straight or branched chain alkyl, benzyl or cyclohexylethyl; or J and K are taken together to form a 7 membered heterocyclic ring which is substituted with oxygen, sulfur, SO, or SO₂.

3. Use of claim 1, wherein said compound is of formula IV 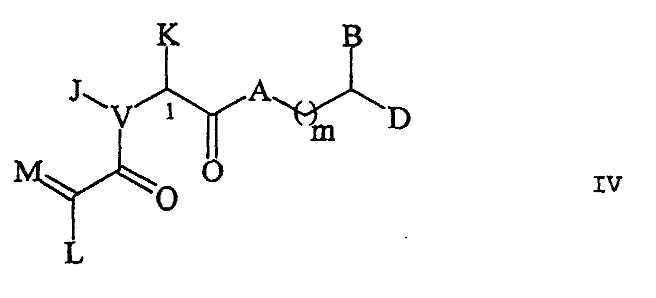 or a pharmaceutically acceptable salt, ester, or solvate thereof, wherein:
V is C, N, or S;
J and K, taken together with V and the carbon atom to which they are respectively attached, form a 7 membered saturated or unsaturated heterocyclic ring having, in addition to V, one or more heteroatom(s) selected from the group consisting of O, S, SO, SO₂, N, NH, and NR;
R is either C₁-C₉ straight or branched chain alkyl, C₂-C₉ straight or branched chain alkenyl, C₃-C₉ cycloalkyl, C₅-C₇ cycloalkenyl, or Ar₁,
wherein R is either unsubstituted of substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₂;
Ar₁ and Ar₂ are independently an alicyclic or aromatic, mono-, bi- or tricyclic, carbo- or heterocyclic ring,
wherein the individual ring size is 5-8 members,
and wherein said heterocyclic ring has 1-6 heteroatom(s) independently selected from the group consisting of O, N, and S;
A is O, NH, or N-(C₁-C₄ alkyl) ;
B and D are independently Ar, C₅-C₇ cycloalkyl substituted C₁-C₆ straight or branched chain alkyl or C₂-C₆ straight or branched chain alkenyl, C₅-C₇ cycloalkenyl substituted C₁-C₆ straight or branched chain alkyl or C₂-C₆ straight or branched chain alkenyl, or Ar substituted C₁-C₆ straight or branched chain alkyl or C₂-C₆ straight or branched chain alkenyl,
wherein in each case, one or two carbon atom(s) of said alkyl or alkenyl is/are optionally substituted with one or two heteroatom(s) independently selected from the group consisting of oxygen, sulfur, SO, and SO₂,
or B and D are independently the fragment 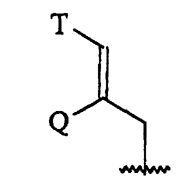 wherein Q is hydrogen, C₁-C₆ straight or branched chain alkyl or C₂-C₆ straight or branched chain alkenyl,
and wherein T is Ar or C₅-C₇ cycloalkyl substituted at positions 3 and 4 with substituents independently selected from the group consisting of hydrogen, hydroxy, O-(C₁-C₄ alkyl) , O-(C₂-C₄ alkenyl), and carbonyl;
Ar is selected from the group consisting of 1-napthyl, 2-napthyl, 2-furyl, 3-furyl, 2-thienyl, 3-thienyl, 2-pyridyl, 3-pyridyl, 4-pyridyl, phenyl, and monocyclic and bicyclic heterocyclic ring systems with individual ring sizes being 5 or 6 which have in either or both rings a total of 1-4 heteroatoms independently selected from oxygen, nitrogen and sulfur,
wherein Ar has 1-3 substituent(s) independently selected from the group consisting of hydrogen, halo, hydroxy, hydroxymethyl, nitro, CF₃, trifluoromethoxy, C₁-C₆ straight or branched chain alkyl, C₂-C₆ straight or branched chain alkenyl, O-(C₁-C₄ straight or branched chain alkyl) , O-(C₂-C₄ straight or branched chain alkenyl), O-benzyl, O-phenyl, amino, 1,2-methylenedioxy, carbonyl, and phenyl;
L is either hydrogen or U, and M is either oxygen or CH-U,
provided that if L is hydrogen, then M is CH-U, or if M is oxygen then L is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇ cycloalkyl, C₅-C₇ cycloalkenyl substituted with C₁-C₄ straight or branched chain alkyl or C₂-C₄ straight or branched chain alkenyl, (C₁-C₄ alkyl or C₂-C₄ alkenyl) -Ar, or Ar; and
m is 0-3.

4. The use of any one of claims 1-3, wherein the compound has an affinity for an FKBP-type immunophilin.

5. The use of claim 4, wherein the FKBP-type immunophilin is FKBP-12.

6. The use of any one of claims 1-3, wherein the compound is immunosuppressive.

7. The use of any one of claims 1-3, wherein the compound is non-immunosuppressive.

8. A pharmaceutical composition which comprises:
(i) an effective amount of a compound for treating alopecia or promoting hair growth in an animal, wherein said compound is of formula IV 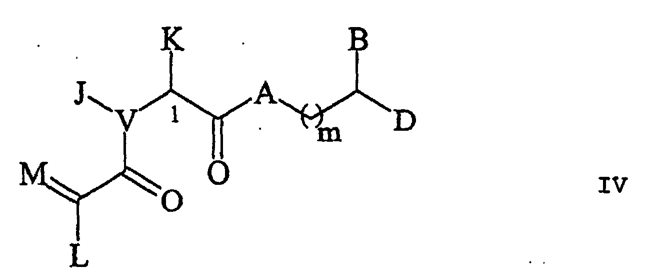 or a pharmaceutically acceptable salt, ester, or solvate thereof, wherein:
V is C, N, or S;
J and K, taken together with V and the carbon atom to which they are respectively attached, form a 7 membered saturated or unsaturated heterocyclic ring having, in addition to V, one or more heteroatom(s) selected from the group consisting of O, S, SO, SO₂, N, NH, and NR;
R is either C₁-C₉ straight or branched chain alkyl, C₂-C₉ straight or branched chain alkenyl, C₃-C₉ cycloalkyl, C₅-C₇ cycloalkenyl, or Ar₁,
wherein R is either unsubstituted of substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₂;
Ar₁ and Ar₂ are independently an alicyclic or aromatic, mono-, bi- or tricyclic, carbo- or heterocyclic ring,
wherein the individual ring size is 5-8 members,
and wherein said heterocyclic ring has 1-6. heteroatom(s) independently selected from the group consisting of O, N, and S;
A is O, NH, or N-(C₁-C₄ alkyl);
B and D are independently Ar, C₅-C₇ cycloalkyl substituted C₁-C₆ straight or branched chain alkyl or C₂-C₆ straight or branched chain alkenyl, C₅-C₇ cycloalkenyl substituted C₁-C₆ straight or branched chain alkyl or C₂-C₆ straight or branched chain alkenyl, or Ar substituted C₁-C₆ straight or branched chain alkyl or C₂-C₆ straight or branched chain alkenyl,
wherein in each case, one or two carbon atom(s) of said alkyl or alkenyl is/are optionally substituted with one or two heteroatom(s) independently selected from the group consisting of oxygen, sulfur, SO, and SO₂,
or B and D are independently the fragment 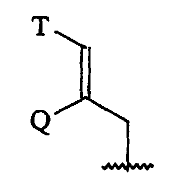 wherein Q is hydrogen, C₁-C₆ straight or branched chain alkyl or C₂-C₆ straight or branched chain alkenyl,
and wherein T is Ar or C₅-C₇ cycloalkyl substituted at positions 3 and 4 with substituents independently selected from the group consisting of hydrogen, hydroxy, O-(C₁-C₄ alkyl) , O-(C₂-C₄ alkenyl), and carbonyl;
Ar is selected from the group consisting of 1-napthyl, 2-napthyl, 2-furyl, 3-furyl, 2-thienyl, 3-thienyl, 2-pyridyl, 3-pyridyl, 4-pyridyl, phenyl, and monocyclic and bicyclic heterocyclic ring systems with individual ring sizes being 5 or 6 which have in either or both rings a total of 1-4 heteroatoms independently selected from oxygen, nitrogen and sulfur,
wherein Ar has 1-3 substituent(s) independently selected from the group consisting of hydrogen, halo, hydroxy, hydroxymethyl, nitro, CF₃, trifluoromethoxy, C₁-C₆ straight or branched chain alkyl, C₂-C₆ straight or branched chain alkenyl, O-(C₁-C₄ straight or branched chain alkyl) , O-(C₂-C₄ straight or branched chain alkenyl), O-benzyl, O-phenyl, amino, 1,2-methylenedioxy, carbonyl, and phenyl;
L is either hydrogen or U, and M is either oxygen or CH-U,
provided that if L is hydrogen, then M is CH-U, or if M is oxygen then L is U;
U is hydrogen, O-(C₁-C₄ straight or branched chain alkyl), O-(C₂-C₄ straight or branched chain alkenyl), C₁-C₆ straight or branched chain alkyl, C₂-C₆ straight or branched chain alkenyl, C₅-C₇ cycloalkyl, C₅-C₇ cycloalkenyl substituted with C₁-C₄ straight or branched chain alkyl or C₂-C₄ straight or branched chain alkenyl, (C₁-C₄ alkyl or C₂-C₄ alkenyl)-Ar, or Ar; and
m is 0-3; and
(ii) a pharmaceutically acceptable carrier.

9. The pharmaceutical composition of claim 8, wherein the compound has an affinity for an FKBP-type immunophilin.

10. The pharmaceutical composition of claim 9, wherein the FKBP-type immunophilin is FKBP-12.

11. The pharmaceutical composition of claim 8, wherein the compound is non-immunosuppressive.

12. The pharmaceutical composition of claim 8, wherein the compound is immunosuppressive.

## Patentansprüche

1. Verwendung einer Verbindung zur Herstellung eines Medikaments zur Behandlung von Alopezie oder zur Förderung des Haarwachstums bei einem dafür bedürftigen Tier, worin die Verbindung eine Verbindung der Formel (I): 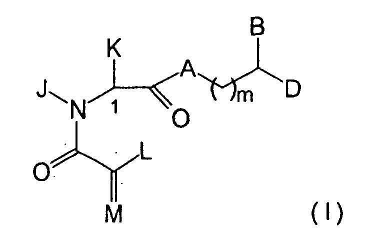 oder ein pharmazeutisch annehmbare(s/r) Salz, Ester oder Solvat davon ist, worin:
A O, NH oder N-(C₁₋₄-Alkyl) ist;
B und D sind unabhängig voneinander Ar, C₅₋₇-Cycloalkyl, substituiertes unverzweigtes oder verzweigtes C₁₋₆-Alkyl oder unverzweigtes oder verzweigtes C₂₋₆-Alkenyl, C₅₋₇-Cycloalkenylsubstituiertes unverzweigtes oder verzweigtes C₁₋₆-Alkyl oder unverzweigtes oder verzweigtes C₂₋₆-Alkenyl, oder Ar-substituiertes, unverzweigtes oder verzweigtes C₁₋₆-Alkyl oder unverzweigtes oder verzweigtes C₂₋₆-Alkenyl, worin in jedem Fall ein oder zwei Kohlenstoffatome des Alkyl- oder Alkenylrests gegebenenfalls mit ein oder zwei Heteroatomen substituiert sind, die unabhängig ausgewählt sind aus Sauerstoff, Schwefel, SO und SO₂,
oder B und D repräsentieren unabhängig voneinander das folgende Fragment: 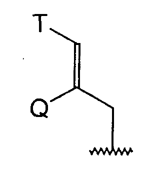 worin Q Wasserstoff, unverzweigtes oder verzweigtes C₁₋₆-Alkyl, unverzweigtes oder verzweigtes C₂₋₆-Alkenyl ist, und worin T Ar oder C₅₋₇-Cycloalkyl ist, das in den Positionen 3 und 4 mit Substituenten, die unabhängig voneinander ausgewählt sind aus Wasserstoff, Hydroxy, O-(C₁₋₄-Alkyl), O-(C₂₋₄-Alkenyl) und Carbonyl, substituiert ist;
Ar ist ausgewählt aus 1-Naphthyl, 2-Naphthyl, 2-Furyl, 3-Furyl, 2-Thienyl, 3-Thienyl, 2-Pyridyl, 3-Pyridyl, 4-Pyridyl, Phenyl und monocyclischen und bicyclischen heterocyclischen Ringsystemen mit individuellen Ringgrössen von 5 oder 6, die in einem oder beiden Ringen eine Gesamtzahl von 1-4 Heteroatomen aufweisen, die unabhängig ausgewählt sind aus Sauerstoff, Stickstoff und Schwefel, worin Ar 1-3 Substituenten aufweist, die unabhängig voneinander ausgewählt sind aus Wasserstoff, Halogen, Hydroxy, Hydroxymethyl, Nitro, CF₃, Trifluormethoxy, unverzweigtem oder verzweigtem C₁₋₆-Alkyl, unverzweigtem oder verzweigtem C₂₋₆-Alkenyl, O-(unverzweigtem oder verzweigtem C₁₋₄-Alkyl), O-(unverzweigtem oder verzweigtem C₂₋₄-Alkenyl), O-Benzyl, O-Phenyl, Amino, 1,2-Methylendioxy, Carbonyl und Phenyl;
L ist entweder Wasserstoff oder U, und M ist entweder Sauerstoff oder CH-U, mit der Massgabe, dass, wenn L Wasserstoff ist, M CH-U ist oder wenn M Sauerstoff ist, L = U ist;
U ist Wasserstoff, O-(unverzweigtes oder verzweigtes C₁₋₄-Alkyl), O-(unverzweigtes oder verzweigtes C₂₋₄-Alkenyl), unverzweigtes oder verzweigtes C₁₋₆-Alkyl, unverzweigtes oder verzweigtes C₂₋₆-Alkenyl, C₅₋₇-Cycloalkyl, C₅₋₇-Cycloalkenyl, das mit unverzweigtem oder verzweigtem C₁₋₄-Alkyl oder unverzweigtem oder verzweigtem C₂₋₄-Alkenyl substituiert ist, (C₁₋₄-Alkyl oder C₂₋₄-Alkenyl)-Ar oder Ar;
J ist Wasserstoff, C₁- oder C₂-Alkyl oder Benzyl; K ist unverzweigtes oder verzweigtes C₁₋₄-Alkyl, Benzyl oder Cyclohexylmethyl; oder J und K bilden zusammen einen 7-gliedrigen heterocyclischen Ring, der mit Sauerstoff, Schwefel, SO oder SO₂ substituiert ist; und
m ist 0-3.

2. Verwendung gemäss Anspruch 1, worin die Verbindung eine Verbindung der Formel (II): 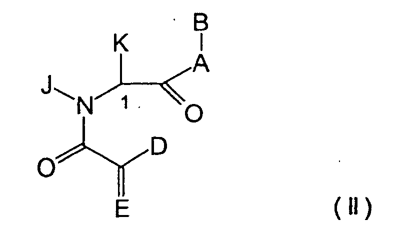 oder ein pharmazeutisch annehmbare(s/r) Salz, Ester oder Solvat davon ist, worin
A O, NH oder N-(C₁₋₄-Alkyl) ist;
B ist CHL-Ar, unverzweigtes oder verzweigtes C₁₋₆-Alkyl, unverzweigtes oder verzweigtes C₂₋₆-Alkenyl, C₅₋₇-Cycloalkyl, C₅₋₇-Cycloalkenyl, Ar-substituiertes C₁₋₆-Alkyl oder C₂₋₆-Alkenyl, oder 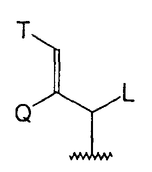 worin L und Q unabhängig voneinander Wasserstoff, unverzweigtes oder verzweigtes C₁₋₆-Alkyl oder unverzweigtes oder verzweigtes C₂₋₆-Alkenyl sind und worin T Ar oder C₅₋₇-Cyclohexyl ist, das in den Positionen 3 und 4 mit Substituenten, die unabhängig voneinander aus Wasserstoff, Hydroxy, O-(C₁₋₄-Alkyl), O-(C₂₋₄-Alkenyl) und Carbonyl ausgewählt sind, substituiert ist;
Ar ist ausgewählt aus 1-Naphthyl, 2-Naphthyl, 2-Furyl, 3-Furyl, 2-Thienyl, 2-Pyridyl, 3-Pyridyl, 4-Pyridyl und Phenyl, worin Ar 1-3 Substituenten aufweist, die unabhängig voneinander ausgewählt sind aus Wasserstoff, Halogen, Hydroxy, Nitro, CF₃, unverzweigtem oder verzweigtem C₁₋₆-Alkyl, unverzweigtem oder verzweigtem C₂₋₆-Alkenyl, O-(unverzweigtem oder verzweigtem C₁₋₄-Alkyl), O-(unverzweigtem oder verzweigtem C₂₋₄-Alkenyl), O-Benzyl, O-Phenyl, Amino und Phenyl;
D ist Wasserstoff oder U und E ist Sauerstoff oder CH-U, mit der Massgabe, dass, wenn D Wasserstoff ist, E CH-U ist oder wenn E Sauerstoff ist, dann ist D = U;
U ist Wasserstoff, O-(unverzweigtes oder verzweigtes C₁₋₄-Alkyl), O-(unverzweigtes oder verzweigtes C₂₋₄-Alkenyl), unverzweigtes oder verzweigtes C₁₋₆-Alkyl, unverzweigtes oder verzweigtes C₂₋₆-Alkenyl, C₅₋₇-Cycloalkyl, C₅₋₇-Cycloalkenyl, das mit unverzweigtem oder verzweigtem C₁₋₄-Alkyl oder unverzweigtem oder verzweigtem C₂₋₄-Alkenyl substituiert ist, 2-Indolyl, 3-Indolyl, (C₁₋₄-Alkyl oder C₂₋₄-Alkenyl)-Ar oder Ar; und
J ist Wasserstoff, C₁- oder C₂-Alkyl oder Benzyl; K ist unverzweigtes oder verzweigtes C₁₋₄-Alkyl, Benzyl oder Cyclohexylethyl; oder J und K bilden zusammen einen 7-gliedrigen heterocyclischen Ring, der mit Sauerstoff, Schwefel, SO oder SO₂ substituiert ist.

3. Verwendung gemäss Anspruch 1, worin die Verbindung eine Verbindung der Formel (IV): 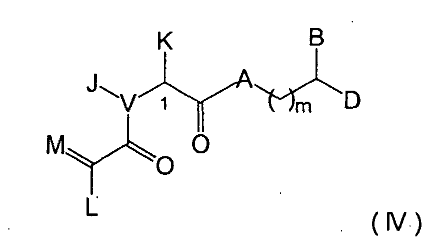 oder ein pharmazeutisch annehmbare(s/r) Salz, Ester oder Solvat davon ist, worin
V C, N oder S ist;
J und K bilden zusammen mit V und dem Kohlenstoffatom, an das sie jeweils gebunden sind, einen 7-gliedrigen, gesättigten oder ungesättigten, heterocyclischen Ring, der zusätzlich zu V ein oder mehrere Heteroatome aufweist, ausgewählt aus O, S, SO, SO₂, N, NH und NR;
R ist entweder unverzweigtes oder verzweigtes C₁₋₉-Alkyl, unverzweigtes oder verzweigtes C₂₋₉-Alkenyl, C₃₋₉-Cycloalkyl, C₅₋₇-Cycloalkenyl oder Ar₁, worin R entweder unsubstituiert ist oder mit einem oder mehreren Substituenten, die unabhängig voneinander ausgewählt sind aus Halogen, Halogenalkyl, Carbonyl, Carboxy, Hydroxy, Nitro, Trifluormethyl, unverzweigtem oder verzweigtem C₁₋₆-Alkyl, unverzweigtem oder verzweigtem C₂₋₆-Alkenyl, C₁₋₄-Alkoxy, C₂₋₄-Alkenyloxy, Phenoxy, Benzyloxy, Thioalkyl, Alkylthio, Sulfhydryl, Amino, Alkylamino, Aminoalkyl, Aminocarboxyl und Ar₂, substituiert ist;
Ar₁ und Ar₂ sind unabhängig voneinander ein alicyclischer oder aromatischer mono-, bi- oder tricyclischer carbo- oder heterocyclischer Ring, worin die individuelle Ringgrösse 5-8 Glieder beträgt und worin der heterocyclische Ring 1-6 Heteroatome aufweist, die unabhängig voneinander ausgewählt sind aus O, N und S;
A ist O, NH oder N-(C₁₋₄-Alkyl);
B und D sind unabhängig voneinander Ar, C₅₋₇-Cycloalkyl-substituiertes, unverzweigtes oder verzweigtes C₁₋₆-Alkyl oder unverzweigtes oder verzweigtes C₂₋₆-Alkenyl, C₅₋₇-Cycloalkenylsubstituiertes, unverzweigtes oder verzweigtes C₁₋₆-Alkyl oder unverzweigtes oder verzweigtes C₂₋₆-Alkenyl, oder Ar-substituiertes, unverzweigtes oder verzweigtes C₁₋₆-Alkyl oder unverzweigtes oder verzweigtes C₂₋₆-Alkenyl, worin in jedem Fall ein oder zwei Kohlenstoffatome des Alkyl- oder Alkenylrests gegebenenfalls mit ein oder zwei Heteroatomen substituiert sind, die unabhängig ausgewählt sind aus Sauerstoff, Schwefel, SO und SO₂,
oder B und D repräsentieren unabhängig voneinander das folgende Fragment: 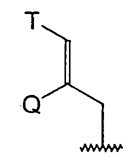 worin Q Wasserstoff, unverzweigtes oder verzweigtes C₁₋₆-Alkyl, oder unverzweigtes oder verzweigtes C₂₋₆-Alkenyl ist, und worin T Ar oder C₅₋₇-Cycloalkyl ist, das in den Positionen 3 und 4 mit Substituenten, die unabhängig voneinander ausgewählt sind aus Wasserstoff, Hydroxy, O-(C₁₋₄-Alkyl), O-(C₂₋₄-Alkenyl) und Carbonyl, substituiert ist;
Ar ist ausgewählt aus 1-Naphthyl, 2-Naphthyl, 2-Furyl, 3-Furyl, 2-Thienyl, 3-Thienyl, 2-Pyridyl, 3-Pyridyl, 4-Pyridyl, Phenyl und monocyclischen und bicyclischen heterocyclischen Ringsystemen mit individuellen Ringgrössen von 5 oder 6, die in einem oder beiden Ringen eine Gesamtzahl von 1-4 Heteroatomen aufweisen, die unabhängig ausgewählt sind aus Sauerstoff, Stickstoff und Schwefel, worin Ar 1-3 Substituenten aufweist, die unabhängig voneinander ausgewählt sind aus Wasserstoff, Halogen, Hydroxy, Hydroxymethyl, Nitro, CF₃, Trifluormethoxy, unverzweigtem oder verzweigtem C₁₋₆-Alkyl, unverzweigtem oder verzweigtem C₂₋₆-Alkenyl, O-(unverzweigtem oder verzweigtem C₁₋₄-Alkyl), O-(unverzweigtem oder verzweigtem C₂₋₄-Alkenyl), O-Benzyl, O-Phenyl, Amino, 1,2-Methylendioxy, Carbonyl und Phenyl;
L ist entweder Wasserstoff oder U und M ist entweder Sauerstoff oder CH-U, mit der Massgabe, dass, wenn L Wasserstoff ist, M CH-U ist oder wenn M Sauerstoff ist, L = U ist;
U ist Wasserstoff, O-(unverzweigtes oder verzweigtes C₁₋₄-Alkyl), O-(unverzweigtes oder verzweigtes C₂₋₄-Alkenyl), unverzweigtes oder verzweigtes C₁₋₆-Alkyl, unverzweigtes oder verzweigtes C₂₋₆-Alkenyl, C₅₋₇-Cycloalkyl, C₅₋₇-Cycloalkenyl, das mit unverzweigtem oder verzweigtem C₁₋₄-Alkyl oder unverzweigtem oder verzweigtem C₂₋₄-Alkenyl substituiert ist, (C₁₋₄-Alkyl oder C₂₋₄-Alkenyl)-Ar oder Ar;
und m ist 0-3.

4. Verwendung gemäss mindestens einem der Ansprüche 1 bis 3, worin die Verbindung eine Affinität gegenüber Immunophilin vom FKBP-Typ aufweist.

5. Verwendung gemäss Anspruch 4, worin das Immunophilin vom FKBP-Typ FKBP-12 ist.

6. Verwendung gemäss mindestens einem der Ansprüche 1 bis 3, worin die Verbindung immunosuppressiv ist.

7. Verwendung gemäss mindestens einem der Ansprüche 1 bis 3, worin die Verbindung nicht-immunosuppressiv ist.

8. Pharmazeutische Zusammensetzung, die folgendes umfasst:
(i) eine wirksame Menge einer Verbindung zur Behandlung von Alopezie oder zur Förderung des Haarwachstums bei einem Tier, worin die Verbindung eine Verbindung der Formel (IV): 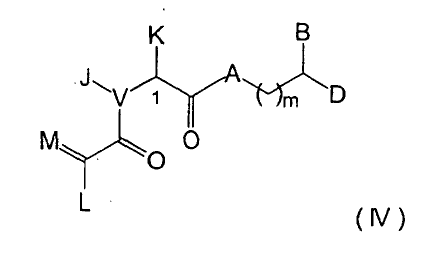 oder ein pharmazeutisch annehmbare(s/r) Salz, Ester oder Solvat davon ist, worin
V C, N oder S ist;
J und K bilden zusammen mit V und dem Kohlenstoffatom, an das sie jeweils gebunden sind, einen 7-gliedrigen, gesättigten oder ungesättigten, heterocyclischen Ring, der zusätzlich zu V ein oder mehrere Heteroatome aufweist, ausgewählt aus O, S, SO, SO₂, N, NH und NR;
R ist entweder unverzweigtes oder verzweigtes C₁₋₉-Alkyl, unverzweigtes oder verzweigtes C₂₋₉-Alkenyl, C₃₋₉-Cycloalkyl, C₅₋₇-Cycloalkenyl oder Ar₁, worin R entweder unsubstituiert ist oder mit einem oder mehreren Substituenten, die unabhängig voneinander ausgewählt sind aus Halogen, Halogenalkyl, Carbonyl, Carboxy, Hydroxy, Nitro, Trifluormethyl, unverzweigtem oder verzweigtem C₁₋₆-Alkyl, unverzweigtem oder verzweigtem C₂₋₆-Alkenyl, C₁₋₄-Alkoxy, C₂₋₄-Alkenyloxy, Phenoxy, Benzyloxy, Thioalkyl, Alkylthio, Sulfhydryl, Amino, Alkylamino, Aminoalkyl, Aminocarboxyl und Ar₂, substituiert ist;
Ar₁ und Ar₂ sind unabhängig voneinander ein alicyclischer oder aromatischer mono-, bi- oder tricyclischer carbo- oder heterocyclischer Ring, worin die individuelle Ringgrösse 5-8 Glieder beträgt und worin der heterocyclische Ring 1-6 Heteroatome aufweist, die unabhängig voneinander ausgewählt sind aus O, N und S;
A ist O, NH oder N-(C₁₋₄-Alkyl);
B und D sind unabhängig voneinander Ar, C₅₋₇-Cycloalkyl-substituiertes, unverzweigtes oder verzweigtes C₁₋₆-Alkyl oder unverzweigtes oder verzweigtes C₂₋₆-Alkenyl, C₅₋₇-Cycloalkenylsubstituiertes, unverzweigtes oder verzweigtes C₁₋₆-Alkyl oder unverzweigtes oder verzweigtes C₂₋₆-Alkenyl, oder Ar-substituiertes, unverzweigtes oder verzweigtes C₁₋₆-Alkyl oder unverzweigtes oder verzweigtes C₂₋₆-Alkenyl, worin in jedem Fall ein oder zwei Kohlenstoffatome des Alkyl- oder Alkenylrests gegebenenfalls mit ein oder zwei Heteroatomen substituiert sind, die unabhängig ausgewählt sind aus Sauerstoff, Schwefel, SO und SO₂,
oder B und D repräsentieren unabhängig voneinander das folgende Fragment: 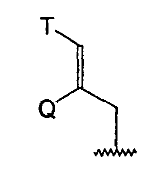 worin Q Wasserstoff, unverzweigtes oder verzweigtes C₁₋₆-Alkyl, unverzweigtes oder verzweigtes C₂₋₆-Alkenyl ist, und worin T Ar oder C₅₋₇-Cycloalkyl ist, das in den Positionen 3 und 4 mit Substituenten, die unabhängig voneinander ausgewählt sind aus Wasserstoff, Hydroxy, O-(C₁₋₄-Alkyl), O-(C₂₋₄-Alkenyl) und Carbonyl, substituiert ist;
Ar ist ausgewählt aus 1-Naphthyl, 2-Naphthyl, 2-Furyl, 3-Furyl, 2-Thienyl, 3-Thienyl, 2-Pyridyl, 3-Pyridyl, 4-Pyridyl, Phenyl und monocyclischen und bicyclischen heterocyclischen Ringsystemen mit individuellen Ringgrössen von 5 oder 6, die in einem oder beiden Ringen eine Gesamtzahl von 1-4 Heteroatomen aufweisen, die unabhängig ausgewählt sind aus Sauerstoff, Stickstoff und Schwefel, worin Ar 1-3 Substituenten aufweist, die unabhängig voneinander ausgewählt sind aus Wasserstoff, Halogen, Hydroxy, Hydroxymethyl, Nitro, CF₃, Trifluormethoxy, unverzweigtem oder verzweigtem C₁₋₆-Alkyl, unverzweigtem oder verzweigtem C₂₋₆-Alkenyl, O-(unverzweigtem oder verzweigtem C₁₋₄-Alkyl), O-(unverzweigtem oder verzweigtem C₂₋₄-Alkenyl), O-Benzyl, O-Phenyl, Amino, 1,2-Methylendioxy, Carbonyl und Phenyl;
L ist entweder Wasserstoff oder U und M ist entweder Sauerstoff oder CH-U, mit der Massgabe, dass, wenn L Wasserstoff ist, M CH-U ist, oder wenn M Sauerstoff ist, L = U ist;
U ist Wasserstoff, O-(unverzweigtes oder verzweigtes C₁₋₄-Alkyl), O-(unverzweigtes oder verzweigtes C₂₋₄-Alkenyl), unverzweigtes oder verzweigtes C₁₋₆-Alkyl, unverzweigtes oder verzweigtes C₂₋₆-Alkenyl, C₅₋₇-Cycloalkyl, C₅₋₇-Cycloalkenyl, das mit unverzweigtem oder verzweigtem C₁₋₄-Alkyl oder unverzweigtem oder verzweigtem C₂₋₄-Alkenyl substituiert ist, (C₁₋₄-Alkyl oder C₂₋₄-Alkenyl)-Ar oder Ar;
und m ist 0-3; und
(ii) einen pharmazeutisch annehmbaren Träger.

9. Pharmazeutische Zusammensetzung gemäss Anspruch 8, worin die Verbindung eine Affinität gegenüber Immunophilin vom FKBP-Typ aufweist.

10. Pharmazeutische Zusammensetzung gemäss Anspruch 9, worin das Immunophilin vom FKBP-Typ FKBP-12 ist.

11. Pharmazeutische Zusammensetzung gemäss Anspruch 8, worin die Verbindung nicht immunosuppressiv ist.

12. Pharmazeutische Zusammensetzung gemäss Anspruch 8, worin die Verbindung immunosuppressiv ist.

## Revendications

1. Utilisation d'un composé pour la préparation d'un médicament destiné au traitement de l'alopécie ou à l'activation de la pousse des poils chez un animal en ayant besoin, dans laquelle ledit composé est un composé de formule I 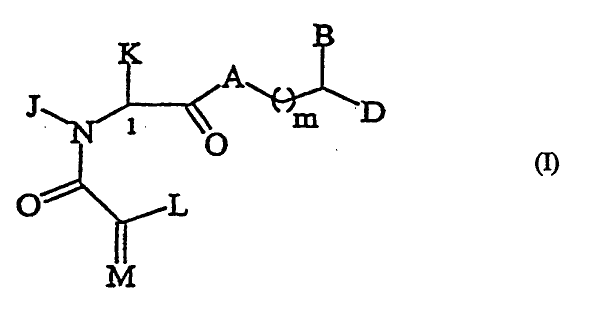 ou un de ses sels, esters ou solvates pharmaceutiquement acceptables, où:
A est O, NH ou N-(alkyle C₁-C₄) ;
B et D sont indépendamment Ar, un alcényle linéaire ou ramifié C₂-C₆ ou alkyle linéaire ou ramifié C₁-C₆ substitué par cycloalkyle C₅-C₇, un alcényle linéaire ou ramifié C₂-C₆ ou alkyle linéaire ou ramifié C₁-C₆ substitué par cycloalcényle C₅-C₇,
ou un alcényle linéaire ou ramifié C₂-C₆ ou alkyle linéaire ou ramifié C₁-C₆ substitué par Ar,
dans lesquels, dans chaque cas, un ou deux atome(s) de carbone dudit alkyle ou alcényle est/sont éventuellement substitué(s) par un ou deux hétéroatome(s) choisis indépendamment dans le groupe consistant en l'oxygène, le soufre, SO et SO₂,
ou B et D représentent indépendamment le fragment 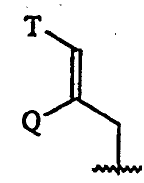 dans lequel Q est l'hydrogène ou un alcényle linéaire ou ramifié C₂-C₆ ou alkyle linéaire ou ramifié C₁-C₆, et dans lequel
T est un Ar ou un cycloalkyle C₅-C₇ substitué aux positions 3 et 4 par des substituants choisis indépendamment dans le groupe consistant en l'hydrogène et l'hydroxy, O-(alkyle C₁-C₄), O-(alcényle C₂-C₄) et carbonyle ;
Ar est choisi dans le groupe consistant en les 1-naphtyle, 2-naphtyle, 2-furyle, 3-furyle, 2-thiényle, 3-thiényle, 2-pyridyle, 3-pyridyle, 4-pyridyle, phényle, et des systèmes cycliques hétérocycliques bicycliques et monocycliques dont les tailles des. cycles individuels sont de 5 ou 6 (chaînons), et qui ont en tout, dans l'un des cycles ou dans les deux, 1 à 4 hétéroatomes choisis indépendamment parmi l'oxygène, l'azote et le soufre,
Ar ayant 1 à 3 substituant(s) choisi(s) indépendamment dans le groupe consistant en l'hydrogène et les groupes halogéno, hydroxy, hydroxyméthyle, nitro, CF₃, trifluorométhoxy, alkyle linéaire ou ramifié C₁-C₆, alcényle linéaire ou ramifié C₂-C₆, O-(alkyle linéaire ou ramifié C₁-C₄), O-(alcényle linéaire ou ramifié C₂-C₄), O-benzyle, O-phényle, amino, 1,2-méthylènedioxy, carbonyle et phényle ;
L est l'hydrogène ou U, et M est l'oxygène ou CH-U, à condition que M soit CH-U lorsque L représente l'hydrogène, ou que L soit U lorsque M représente l'oxygène;
U représente l'hydrogène ou un groupe O-(alkyle linéaire ou ramifié C₁-C₄), O-(alcényle linéaire ou ramifié C₂-C₄), alkyle linéaire ou ramifié C₁-C₆, alcényle linéaire ou ramifié C₂-C₆, cycloalkyle C₅-C₇, cycloalcényle C₅-C₇ substitué par alkyle linéaire ou ramifié C₁-C₄ ou par alcényle linéaire ou ramifié C₂-C₄, (alkyl C₁-C₄ ou alcényl C₂-C₄)-Ar, ou Ar ;
J représente l'hydrogène ou un alkyle C₁ ou C₂, ou benzyle ;
K représente un alkyle linéaire ou ramifié C₁-C₄, benzyle ou cyclohexylméthyle ;
ou J et K sont pris ensemble pour former un cycle hétérocyclique de 7 chaînons substitué par l'oxygène, le soufre, SO ou SO₂ ; et
m est 0-3.

2. Utilisation selon la revendication 1, dans laquelle ledit composé est un composé de formule II 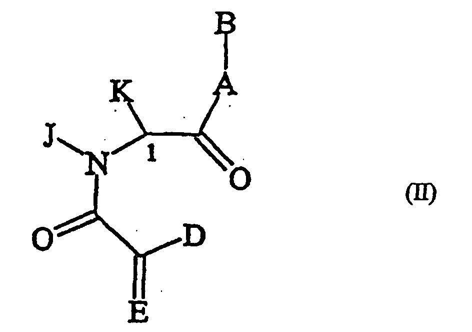 ou un de ses sels, esters ou solvates pharmaceutiquement acceptables, où:
A est O, NH ou N-(alkyle en C₁-C₄) ;
B représente l'hydrogène, CHL-Ar, un alkyle linéaire ou ramifié C₁-C₆, un alcényle linéaire ou ramifié C₂-C₆, un cycloalkyle C₅-C₇, un cycloalcényle C₅-C₇, un alcényle C₂-C₆ ou alkyle C₁-C₆ substitué Ar, ou 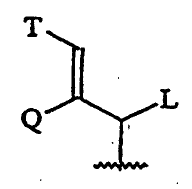 dans lequel L et Q représentent indépendamment l'hydrogène, un alkyle linéaire ou ramifié C₁-C₆, un alcényle linéaire ou ramifié C₂-C₆, et dans lequel
T est un Ar ou cyclohexyle C₅-C₇ substitué aux positions 3 et 4 par des substituants choisis indépendamment dans le groupe consistant en l'hydrogène et les hydroxy, O-(alkyle C₁-C₄), O-(alcényle C₂-C₄) et carbonyle ;
Ar est choisi dans le groupe consistant en les 1-naphtyle, 2-naphtyle, 2-furyle, 3-furyle, 2-thiényle, 2-pyridyle, 3-pyridyle, 4-pyridyle et phényle, où Ar a 1 à 3 substituants choisis indépendamment dans le groupe consistant en l'hydrogène et les halogéno, hydroxy, nitro, CF₃, alkyle linéaire ou ramifié C₁-C₆, alcényle linéaire ou ramifié C₂-C₆, O-(alkyle linéaire ou ramifié C₁-C₄), O-(alcényle linéaire ou ramifié C₂-C₄), O-benzyle, O-phényle, amino et phényle ;
D est l'hydrogène ou U, et E est l'oxygène ou CH-U, à condition que E soit CH-U lorsque D représente l'hydrogène, ou que D soit U lorsque E représente l'oxygène ;
U représente l'hydrogène, un O-(alkyle linéaire ou ramifié C₁-C₄), O-(alcényle linéaire ou ramifié C₂-C₄), alkyle linéaire ou ramifié C₁-C₆, alcényle linéaire ou ramifié C₂-C₆, cycloalkyle C₅-C₇, cycloalcényle C₅-C₇ substitué par alkyle linéaire ou ramifié C₁-C₄ ou par alcényle linéaire ou ramifié C₂-C₄, 2-indolyle, 3-indolyle, (alkyl C₁-C₄ ou alcényl C₁-C₄)-Ar ou Ar ; et
J représente l'hydrogène, un alkyle C₁ ou C₂, ou benzyle ;
K représente un alkyle linéaire ou ramifié C₁-C₄, benzyle ou cyclohexyléthyle ;
ou J et K sont pris ensemble pour former un hétérocycle de 7 chaînons substitué par l'oxygène, le soufre, SO ou SO₂.

3. Utilisation selon la revendication 1, dans laquelle ledit composé est un composé de formule IV 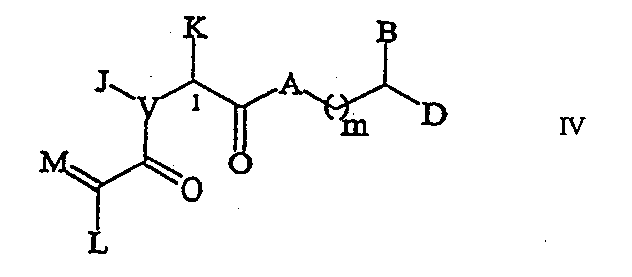 ou un de ses sels, esters ou solvates pharmaceutiquement acceptables, où
V est C, N ou S ;
J et K, pris ensemble avec V et l'atome de carbone auquel ils sont respectivement liés, forment un cycle hétérocyclique saturé ou insaturé de 7 chaînons ayant, en plus de V, un ou plusieurs hétéroatome(s) choisi(s) dans le groupe consistant en O, S, SO, SO₂, N, NH et NR ;
R représente un groupe alkyle linéaire ou ramifié C₁-C₉, alcényle linéaire ou ramifié C₂-C₉, cycloalkyle C₃-C₉, cycloalcényle C₅-C₇ ou Ar₁,
R étant non substitué ou substitué par un ou plusieurs substituant(s) choisi(s) indépendamment dans le groupe consistant en les halogéno, halogénoalkyle, carbonyle, carboxy, hydroxy, nitro, trifluorométhyle, alkyle linéaire ou ramifié C₁-C₆, alcényle linéaire ou ramifié C₂-C₆, alcoxy C₁-C₄, alcényloxy C₂-C₄, phénoxy, benzyloxy, thioalkyle, alkylthio, sulfhydryle, amino, alkylamino, aminoalkyle, aminocarboxyle et Ar₂ ;
Ar₁ et Ar₂ représentent indépendamment un cycle carbo- ou hétérocyclique, mono-, bi- ou tricyclique, alicyclique ou aromatique, la taille des cycles individuels étant 5-8 chaînons, et ledit cycle hétérocyclique ayant 1 à 6 hétéroatome(s) choisi(s) indépendamment dans le groupe consistant en O, N et S;
A est O, NH ou N-(alkyle C₁-C₄);
B et D représentent indépendamment Ar, un alcényle linéaire ou ramifié C₂-C₆ ou alkyle linéaire ou ramifié C₁-C₆ substitué par cycloalkyle C₅-C₇, un alcényle linéaire ou ramifié C₂-C₆ ou alkyle linéaire ou ramifié C₁-C₆ substitué par cycloalcényle C₅-C₇, ou un alcényle linéaire ou ramifié C₂-C₆ ou alkyle linéaire ou ramifié C₁-C₆ substitué par Ar,
dans lesquels, dans chaque cas, un ou deux atome(s) de carbone dudit alkyle ou alcényle est/sont éventuellement substitué(s) par un ou deux hétéroatome(s) choisi(s). indépendamment dans le groupe consistant en l'oxygène, le soufre, SO et SO₂, ou B et D représentent indépendamment le fragment 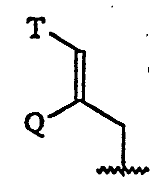 dans lequel Q est l'hydrogène ou un alkyle linéaire ou ramifié C₁-C₆ ou alcényle linéaire ou ramifié C₂-C₆, et dans lequel
T est Ar ou cycloalkyle C₅-C₇ substitué aux positions 3 et 4 par des substituants choisis indépendamment dans le groupe consistant en l'hydrogène et les hydroxy, O-(alkyle C₁-C₄), O-(alcényle C₂-C₄) et carbonyle ;
Ar est choisi dans le groupe consistant en les 1-naphtyle, 2-naphtyle, 2-furyle, 3-furyle, 2-thiényle, 3-thiényle, 2-pyridyle, 3-pyridyle, 4-pyridyle, phényle, et des systèmes cycliques hétérocycliques monocycliques et bicycliques dont les cycles individuels ont une taille de 5 ou 6 (chaînons), et qui ont en tout, dans l'un des cycles ou dans les deux, 1 à 4 hétéroatome(s) choisi(s) indépendamment parmi l'oxygène, l'azote et le soufre,
Ar ayant 1 à 3 substituant(s) choisi(s) indépendamment dans le groupe consistant en l'hydrogène et les halogéno, hydroxy, hydroxyméthyle, nitro, CF₃, trifluorométhoxy, alkyle linéaire ou ramifié C₁-C₆, alcényle linéaire ou ramifié C₂-C₆, O-(alkyle linéaire ou ramifié C₁-C₄), O-(alcényle linéaire ou ramifié C₂-C₄), O-benzyle, O-phényle, amino, 1,2-méthylènedioxy, carbonyle et phényle ;
L est l'hydrogène ou U, et M est l'oxygène ou CH-U, à condition que M soit CH-U lorsque L représente l'hydrogène, ou que L soit U lorsque M représente l'oxygène;
U représente l'hydrogène, un O-(alkyle linéaire ou ramifié C₁-C₄), O-(alcényle linéaire ou ramifié C₂-C₄), alkyle linéaire ou ramifié C₁-C₆, alcényle linéaire ou ramifié C₂-C₆, cycloalkyle C₅-C₇, cycloalcényle C₅-C₇ substitué par alkyle linéaire ou ramifié C₁-C₄ ou par, alcényle linéaire ou ramifié C₂-C₄, (alkyl C₁-C₄ ou alcényl C₂-C₄)-Ar, ou Ar ; et
m est 0-3.

4. Utilisation selon l'une quelconque des revendications 1-3, dans laquelle le composé a une affinité pour une immunophiline de type FKBP.

5. Utilisation selon la revendication 4, dans laquelle l'immunophiline de type FKBP est la FKBP-12.

6. Utilisation selon l'une quelconque des revendications 1-3, dans laquelle le composé est immunosuppresseur.

7. Utilisation selon l'une quelconque des revendications 1-3, dans laquelle le composé n'est pas immunosuppresseur.

8. Composition pharmaceutique qui comprend:
(i) une quantité efficace d'un composé pour le traitement de l'alopécie ou l'activation de la pousse des poils chez un animal, ledit composé étant un composé de formule IV 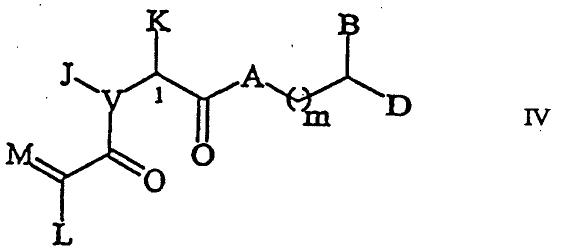 ou un de ses sels, esters ou solvates pharmaceutiquement acceptables, où
V est C, N ou S ;
J et K, pris ensemble avec V et l'atome de carbone auquel ils sont respectivement liés, forment un cycle hétérocyclique saturé ou insaturé de 7 chaînons ayant, en plus de V, un ou plusieurs hétéroatome(s) choisi(s) dans le groupe consistant en O, S, SO, SO₂, N, NH et NR ;
R représente un alkyle linéaire ou ramifié C₁-C₉, alcényle linéaire ou ramifié C₂-C₉, cycloalkyle C₃-C₉, cycloalcényle C₅-C₇, ou Ar₁,
R étant non substitué ou substitué par un ou plusieurs substituant(s) choisi(s) indépendamment dans le groupe consistant en les halogène, halogénoalkyle, carbonyle, carboxy, hydroxy, nitro, trifluorométhyle, alkyle linéaire ou ramifié C₁-C₆, alcényle linéaire ou ramifié C₂-C₆, alcoxy C₁-C₄, alcényloxy C₂-C₄, phénoxy, benzyloxy, thioalkyle, alkylthio, sulfhydryle, amino, alkylamino, aminoalkyle, aminocarboxyle, et Ar₂ ;
Ar₁ et Ar₂ représentent indépendamment un cycle carbo- ou hétérocyclique, mono-, bi- ou tricyclique, alicyclique ou aromatique, la taille des cycles individuels étant 5-8 chaînons, et ledit cycle hétérocyclique ayant 1 à 6 hétéroatome(s) choisi(s) indépendamment dans le groupe consistant en O, N et S ;
A est 0, NH ou N-(alkyle C₁-C₄) ;
B et D représentent indépendamment Ar, un alcényle linéaire ou ramifié C₂-C₆ ou alkyle linéaire ou ramifié C₁-C₆ substitué par cycloalkyle C₅-C₇, un alcényle linéaire
ou ramifié C₂-C₆ ou alkyle linéaire ou ramifié C₁-C₆ substitué par cycloalcényle C₅-C₇, ou un alcényle linéaire ou ramifié C₂-C₆ ou alkyle linéaire ou ramifié C₁-C₆ substitué par Ar,
dans lesquels, dans chaque cas, un ou deux atome(s) de carbone dudit alkyle ou alcényle sont éventuellement substitué(s) par un ou deux hétéroatome(s) choisi(s) indépendamment dans le groupe consistant en l'oxygène, le soufre, SO et SO₂,
ou B et D représentent indépendamment le fragment 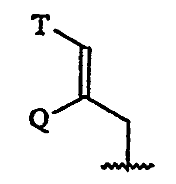 dans lequel Q est l'hydrogène ou un alkyle linéaire ou ramifié C₁-C₆ ou alcényle linéaire ou ramifié C₂-C₆, et dans lequel
T est Ar ou cycloalkyle C₅-C₇ substitué aux positions 3 et 4 par des substituants choisis indépendamment dans le groupe consistant en l'hydrogène et les hydroxy, O-(alkyle C₁-C₄), O-(alcényle C₂-C₄) et carbonyle ;
Ar est choisi dans le groupe consistant en les 1-naphtyle, 2-naphtyle, 2-furyle, 3-furyle, 2-thiényle, 3-thiényle, 2-pyridyle, 3-pyridyle, 4-pyridyle, phényle, et des systèmes cycliques hétérocycliques monocycliques et bicycliques dont les cycles individuels ont une taille de 5 ou 6 (chaînons), et qui ont en tout, dans l'un des cycles ou dans les deux, 1 à 4 hétéroatome(s) choisi(s) indépendamment parmi l'oxygène, l'azote et le soufre,
Ar ayant 1 à 3 substituant(s) choisi(s) indépendamment dans le groupe consistant en l'hydrogène et les halogéno, hydroxy, hydroxyméthyle, nitro, CF₃, trifluorométhoxy, alkyle linéaire ou ramifié C₁-C₆, alcényle linéaire ou ramifié C₂-C₆, O-(alkyle linéaire
ou ramifié C₁-C₄), O-(alcényle linéaire ou ramifié C₂-C₄), O-benzyle, O-phényle, amino, 1,2-méthylènedioxy, carbonyle et phényle ;
L est l'hydrogène ou U, et M est l'oxygène ou CH-U, à condition que M soit CH-U lorsque L représente l'hydrogène, ou que L soit U lorsque M représente l'oxygène;
U représente l'hydrogène, O-(alkyle linéaire ou ramifié C₁-C₄), O-(alcényle linéaire ou ramifié C₂-C₄), alkyle linéaire ou ramifié C₁-C₆, alcényle linéaire ou ramifié C₂-C₆, cycloalkyle C₅-C₇, cycloalcényle C₅-C₇ substitué par alkyle linéaire ou ramifié C₁-C₄ ou par alcényle linéaire ou ramifié C₂-C₄, (alkyl C₁-C₄ ou alcényl C₂-C₄)-Ar,
ou Ar ; et
m est 0-3; et
(ii) un support pharmaceutiquement acceptable.

9. Composition pharmaceutique selon la revendication 8, dans laquelle le composé a une affinité pour une immunophiline de type FKBP.

10. Composition pharmaceutique selon la revendication 9, dans laquelle l'immunophiline de type FKBP est la FKBP-12.

11. Composition pharmaceutique selon la revendication 8, dans laquelle le composé est non immunosuppresseur.

12. Composition pharmaceutique selon la revendication 8, dans laquelle le composé est immunosuppresseur.
